(19) Europäisches Patentamt
European Patent Office
Office européen des brevets

(11) Veröffentlichungsnummer: **0 591 891 A2**

(12) # EUROPÄISCHE PATENTANMELDUNG

(21) Anmeldenummer: **93115991.7**

(22) Anmeldetag: **04.10.93**

(51) Int. Cl.[5]: **C07D 235/08**, C07D 403/10, A61K 31/415

(30) Priorität: **06.10.92 DE 4233590**
**08.05.93 DE 4315349**

(43) Veröffentlichungstag der Anmeldung:
**13.04.94 Patentblatt 94/15**

(84) Benannte Vertragsstaaten:
**AT BE CH DE DK ES FR GB GR IE IT LI LU NL PT SE**

(71) Anmelder: **Dr. Karl Thomae GmbH**

**D-88397 Biberach(DE)**

(72) Erfinder: **Ries, Uwe, Dr. Dipl.-Chem.**
**Dunantstrasse 10**
**D-88400 Biberach(DE)**
Erfinder: **Hauel, Norbert, Dr. Dipl.-Chem.**
**Marderweg 12**
**D-88433 Schemmerhofen(DE)**
Erfinder: **van Meel, Jacques, Dr.**
**Schubertweg 4**
**D-88441 Mittelbiberach(DE)**
Erfinder: **Wienen, Wolfgang, Dr.**
**Am Schiessberg 28**
**D-88437 Äpfingen(DE)**
Erfinder: **Entzeroth, Michael, Dr. Dipl.-Chem.**
**Sebastian-Sailer-Strasse 44**
**D-88447 Warthausen(DE)**

(54) **Benzimidazolderivate als Angiotensin II Antagonisten.**

(57) Die Erfindung betrifft Benzimidazole der allgemeinen Formel

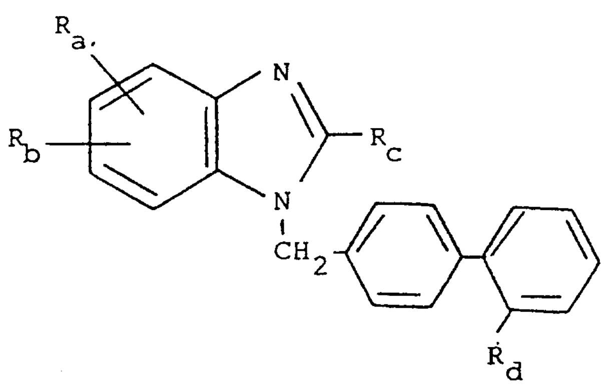

,(I)

in der
$R_a$ bis $R_d$ wie im Anspruch 1 definiert sind, deren 1-, 3-Isomerengemische und deren Salze, welche wertvolle Eigenschaften aufweisen, diese Verbindungen enthaltende Arzeinmittel und deren Verwendung sowie Verfahren zu ihrer Herstellung.

Die neuen Verbindungen stellen insbesondere Angiotensin-Antagonisten dar.



Rank Xerox (UK) Business Services
(3.10/3.09/3.3.4)

Die vorliegende Erfindung betrifft neue Benzimidazole der allgemeinen Formel

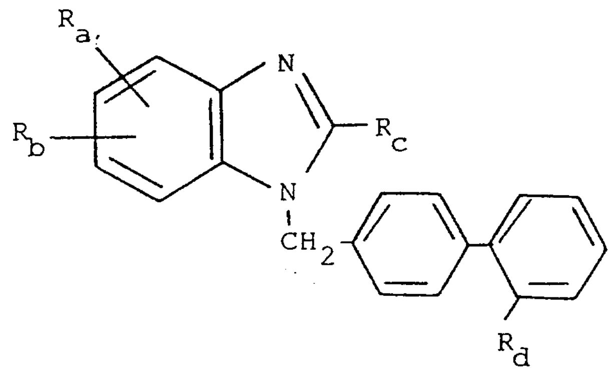

,(I)

deren 1-, 3-Isomerengemische sowie deren Salze, insbesondere für die pharmazeutische Anwendung deren physiologisch verträglichen Salze mit anorganischen oder organischen Säuren oder Basen, welche wertvolle Angiotensin-Antagonisten, insbesondere Angiotensin-II-Antagonisten, darstellen, diese Verbindungen enthaltende Arzneimittel und deren Verwendung sowie Verfahren zu ihrer Herstellung.

In der obigen allgemeinen Formel I bedeutet

$R_a$ eine Alkylgruppe mit 1 bis 3 Kohlenstoffatomen, eine Trifluormethylgruppe, ein Wasserstoff-, Fluor-, Chlor- oder Bromatom,

$R_b$ eine $R_1$NH-Gruppe, in der das Wasserstoffatom durch eine R-CX-Gruppe, durch eine in 2-Stellung durch eine Alkoxygruppe mit 1 bis 3 Kohlenstoffatomen oder durch eine ($R_1NR_3$)-Gruppe substituierte 3,4-Dioxo-1-cyclobuten-1-yl-Gruppe oder durch eine 3-Nitro-pyrrol-2-yl-Gruppe ersetzt ist, wobei die Pyrrolgruppe in 1-Stellung zusätzlich durch eine Alkylgruppe mit 1 bis 3 Kohlenstoffatomen substituiert sein kann, in denen

R eine Alkylgruppe mit 1 bis 5 Kohlenstoffatomen, eine Alkoxy- oder Alkylthiogruppe mit jeweils 1 bis 3 Kohlenstoffatomen, eine Phenoxy- oder Phenylthiogruppe oder eine ($R_2NR_3$)-Gruppe und

X eine $NO_2$-CH =, ($R_4R_5$)C = oder $R_6$-N = Gruppe, in denen

$R_1$ ein Wasserstoffatom oder eine Alkylgruppe mit 1 bis 3 Kohlenstoffatomen,

$R_2$ ein Wasserstoffatom, eine verzweigte oder unverzweigte Alkylgruppe mit 1 bis 10 Kohlenstoffatomen, die in 2-, 3- oder 4-Stellung durch eine Hydroxy-, Alkoxy-, Amino-, Alkylamino- oder Dialkylaminogruppe substituiert sein kann, in denen der Alkylteil jeweils 1 bis 3 Kohlenstoffatome enthalten kann, eine Alkylgruppe mit 1 bis 4 Kohlenstoffatomen, die durch eine Phenyl- oder Pyridylgruppe substituiert ist und zusätzlich in 2-, 3- oder 4-Stellung durch eine Hydroxygruppe substituiert sein kann, eine Cycloalkylgruppe mit 3 oder 4 Kohlenstoffatomen, eine Cycloalkylgruppe mit 5 bis 8 Kohlenstoffatomen, in der eine Ethylenbrücke durch eine o-Phenylengruppe ersetzt sein kann, eine gegebenenfalls durch 1, 2 oder 3 Alkylgruppen substituierte Bicycloalkylgruppe mit 6 bis 8 Kohlenstoffatomen,

$R_3$ ein Wasserstoffatom oder eine Alkylgruppe, die in 2- oder 3-Stellung durch eine Alkoxy-, Amino-, Alkylamino- oder Dialkylaminogruppe substituiert sein kann, wobei die vorstehend erwähnten Alkyl- und Alkoxyteile jeweils 1 bis 3 Kohlenstoffatome enthalten können, oder

$R_2$ und $R_3$ zusammen mit dem dazwischenliegenden Stickstoffatom eine cyclische Alkyleniminogruppe mit 4 bis 6 Kohlenstoffatomen, die durch ein oder zwei Alkylgruppen oder durch eine Phenylgruppe substituiert sein kann und in der zusätzlich eine Ethylenbrücke in 3,4-Stellung durch eine o-Phenylengruppe ersetzt sein kann, eine Morpholinogruppe oder eine gegebenenfalls in 4-Stellung durch eine Alkylgruppe mit 1 bis 3 Kohlenstoffatomen oder durch eine Phenylgruppe substituierte Piperazinogruppe,

$R_4$ und $R_5$, die gleich oder verschieden sein können, jeweils eine Cyano-, $R_2NR_3$-CO- oder Alkoxycarbonylgruppe mit insgesamt 2 bis 5 Kohlenstoffatomen, wobei $R_2$ und $R_3$ wie vorstehend erwähnt definiert sind, und

$R_6$ eine Cyano-, Tetrazol-5-yl-, Alkansulfonyl-, Phenylsulfonyl-, Phenylalkansulfonyl-, Aminosulfonyl-, Alkylaminosulfonyl-, Dialkylaminosulfonyl-, Alkylcarbonyl-, Phenylcarbonyl-, Aminocarbonyl-, Alkylaminocarbonyl- oder Dialkylaminocarbonylgruppe, in denen der Alkylteil jeweils 1 bis 3 Kohlenstoffatome enthalten kann, darstellen,

$R_c$ eine Alkylgruppe mit 2 bis 4 Kohlenstoffatomen, eine Alkoxy- oder Alkylthiogruppe mit jeweils 2 oder 3 Kohlenstoffatomen im Alkylteil, eine Cyclopropyl- oder Cyclobutylgruppe und

$R_d$ eine in vivo in eine Carboxygruppe überführbare Gruppe, eine Carboxy-, Cyano-, 1H-Tetrazolyl-, 1-Triphenylmethyl-te-trazolyl- oder 2-Triphenylmethyl-tetrazolylgruppe.

Unter dem vorstehend erwähnten Begriff "eine Gruppe, die in vivo in eine Carboxygruppe übergeführt wird," sind beispielsweise deren Ester der Formeln

- CO - OR',
- CO - O - (HCR'') - O - CO - R''' und
- CO - O - (HCR'') - O - CO - OR'''

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten, zu verstehen.

Bevorzugte Verbindungen der obigen allgemeinen Formel I sind diejenigen, in denen
$R_a$ eine Methylgruppe, ein Wasserstoff-, Fluor-, Chlor- oder Bromatom,
$R_b$ eine $R_1$NH-Gruppe, in der das Wasserstoffatom durch eine R-CX-Gruppe, durch eine in 2-Stellung durch eine Alkoxygruppe mit 1 bis 3 Kohlenstoffatomen oder durch eine ($R_1NR_3$)-Gruppe substituierte 3,4-Dioxo-1-cyclobuten-1-yl-Gruppe oder durch eine 3-Nitro-pyrrol-2-yl-Gruppe ersetzt ist, wobei die Pyrrolgruppe in 1-Stellung zusätzlich durch eine Methylgruppe substituiert sein kann, in denen

R eine Alkylgruppe mit 1 bis 3 Kohlenstoffatomen, eine Methoxy-, Methylthio-, Phenoxy- oder Phenylthiogruppe oder eine ($R_2NR_3$)-Gruppe und

X eine $NO_2$-CH=, $(NC)_2C$= oder $R_6$-N= Gruppe, in denen

$R_1$ ein Wasserstoffatom oder eine Alkylgruppe mit 1 bis 3 Kohlenstoffatomen,

$R_2$ eine Alkylgruppe mit 1 bis 3 Kohlenstoffatomen, die in 2- oder 3-Stellung durch eine Hydroxy-, Alkoxy-, Amino-, Alkylamino- oder Dialkylaminogruppe substituiert sein kann, wobei der Alkoxy- und Alkylteil jeweils 1 bis 3 Kohlenstoffatome enthalten kann, oder eine Cycloalkylgruppe mit 5 bis 7 Kohlenstoffatomen,

$R_3$ ein Wasserstoffatom, eine Alkylgruppe mit 1 bis 3 Kohlenstoffatomen, eine 2-Methoxyethyl-, 2-Dimethylaminoethyl- oder 2-Diethylaminoethylgruppe oder

$R_2$ und $R_3$ zusammen mit dem dazwischenliegenden Stickstoffatom eine cyclische Alkyleniminogruppe mit 4 oder 5 Kohlenstoffatomen, die durch ein oder zwei Methylgruppen substituiert sein kann, eine Morpholinogruppe oder eine gegebenenfalls in 4-Stellung durch eine Alkylgruppe mit 1 bis 3 Kohlenstoffatome substituierte Piperazinogruppe und

$R_6$ eine Alkylsulfonylgruppe mit 1 bis 3 Kohlenstoffatomen im Alkylteil, eine Cyano-, Aminosulfonyl- oder Phenylsulfonylgruppe darstellen,
$R_c$ eine Alkylgruppe mit 2 bis 4 Kohlenstoffatomen und
$R_d$ eine Carboxy-, 1H-Tetrazolyl-, 1-Triphenylmethyl-te-trazolyl- oder 2-Triphenylmethyl-tetrazolylgruppe bedeuten,
deren 1-, 3-Isomerengemische und deren Salze.

Besonders bevorzugte Verbindungen der obigen allgemeinen Formel I sind diejenigen, in denen
$R_a$ in 4-Stellung eine Methylgruppe oder ein Chloratom,
$R_b$ in 6-Stellung eine $R_1$NH-Gruppe, in der das Wasserstoffatom durch eine R-CX-Gruppe, durch eine in 2-Stellung durch eine ($R_1NR_3$)-Gruppe substituierte 3,4-Dioxo-1-cyclobuten-1-yl-Gruppe oder durch eine 3-Nitro-pyrrol-2-yl-Gruppe ersetzt ist, wobei die Pyrrolgruppe in 1-Stellung zusätzlich durch eine Methylgruppe substituiert sein kann, in denen

R eine ($R_2NR_3$)-Gruppe oder eine Alkylgruppe mit 1 bis 3 Kohlenstoffatomen und

X eine $NO_2$-CH=, $(NC)_2C$= oder $R_6$-N= Gruppe, in denen

$R_1$ ein Wasserstoffatom oder eine Methylgruppe,

$R_2$ eine Alkylgruppe mit 1 bis 3 Kohlenstoffatomen, eine 2-Hydroxyethyl-, 2-Methoxyethyl-, 2-Dimethylaminoethyl- oder 2-Diethylaminoethylgruppe oder eine Cycloalkylgruppe mit 5 bis 7 Kohlenstoffatomen,

$R_3$ ein Wasserstoffatom, eine Alkylgruppe mit 1 bis 3 Kohlenstoffatomen, eine 2-Methoxyethyl-, 2-Dimethylaminoethyl- oder 2-Diethylaminoethylgruppe oder

$R_2$ und $R_3$ zusammen mit dem dazwischenliegenden Stickstoffatom eine Pyrrolidino-, Piperidino-, Morpholino- oder 4-Methyl-piperazinogruppe und

$R_6$ eine Cyano-, Aminosulfonyl-, Methylsulfonyl- oder Phenylsulfonylgruppe darstellen,
$R_c$ eine Alkylgruppe mit 2 bis 4 Kohlenstoffatomen und

$R_d$ eine Carboxy- oder 1H-Tetrazolylgruppe bedeuten,
insbesondere die Verbindungen

(a) 4'-[[2-n-Propyl-4-methyl-6-(2-cyano-3-methyl-guanidino)-1H-benzimidazol-1-yl)-methyl]-biphenyl-2-carbonsäure,

(b) 4'-[[2-n-Propyl-4-methyl-6-(2-cyano-3,3-dimethyl-guanidino)-1H-benzimidazol-1-yl]-methyl]-2-(1H-tetrazol-5-yl)-biphenyl,

(c) 4'-[[2-n-Propyl-4-methyl-6-(2,2-dicyano-1-dimethylaminoethenylenamino)-1H-benzimidazol-1-yl]-methyl]-biphenyl-2-carbonsäure,

(d) 4'-[[2-n-Propyl-4-methyl-6-(1-dimethylamino-2-nitroethenylenamino)-1H-benzimidazol-1-yl]-methyl]-biphenyl-2-carbonsäure,

(e) 4'-[[2-n-Propyl-4-methyl-6-(3-methyl-2-amidosulfonylguanidino)-1H-benzimidazol-1-yl]-methyl]-biphenyl-2-carbonsäure,

(f) 4'-[[2-n-Propyl-4-methyl-6-(1,1-dimethylamino-2-nitroethylenamino)-1H-benzimidazol-1-yl]-methyl]-2-(1H-tetrazol-5-yl)-biphenyl,

(g) 4'-[[2-Ethyl-4-methyl-6-[(2-dimethylamino-3,4-dioxo-1-cyclobuten-1-yl)-amino]-1H-benzimidazol-1-yl]-methyl]-biphenyl-2-carbonsäure und

(h) 4'-[[2-Ethyl-4-methyl-6-(3,3-dimethyl-2-methylsulfonylguanidino)-1H-benzimidazol-1-yl]-methyl]-2-(1H-tetrazol5-yl)-biphenyl,

deren 1-, 3-Isomerengemische und deren Salze.

Erfindungsgemäß erhält man die Verbindungen nach folgenden Verfahren:

a) Zur Herstellung von Verbindungen der allgemeinen Formel I, in der R eine Alkoxy- oder Alkylthiogruppe mit jeweils 1 bis 3 Kohlenstoffatomen oder eine Phenyloxy- oder Phenylthiogruppe darstellt:
Umsetzung einer Verbindung der allgemeinen Formel

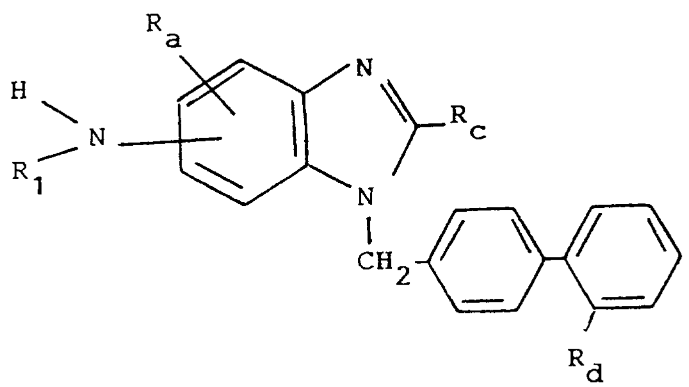

,(II)

in der
$R_a$, $R_c$, $R_d$ und $R_1$ wie eingangs definiert sind, mit einer Verbindung der allgemeinen Formel

$(Y')_2C=X$ ,(III)

in der
X wie eingangs definiert ist,
die Reste Y', die gleich oder verschieden sein können, jeweils eine Alkoxy- oder Alkylthiogruppe mit jeweils 1 bis 3 Kohlenstoffatomen, eine Phenoxy- oder Phenylthiogruppe darstellen.

Die Umsetzung wird vorzugsweise in einem Lösungsmittel wie Methanol, Äthanol, Isopropanol, Dioxan, Tetrahydrofuran oder Chloroform gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50°C, vorzugsweise jedoch bei Raumtemperatur, durchgeführt.

b) Zur Herstellung von Verbindungen der allgemeinen Formel I, in der R eine $(R_2NR_3)$-Gruppe darstellt:
Umsetzung einer Verbindung der allgemeinen Formel

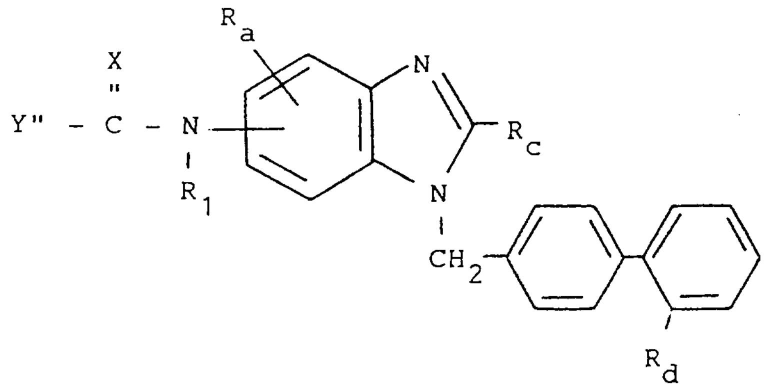

,(IV)

in der
$R_a$, $R_c$, $R_d$, $R_1$ und X wie eingangs definiert sind und
Y" eine Alkoxy-, Alkylthio-, Alkylsulfinyl- oder Alkylsulfonylgruppe mit jeweils 1 bis 3 Kohlenstoffatomen, eine Phenyloxy-, Phenylthio-, Phenylsulfinyl- oder Phenylsulfonylgruppe darstellt, mit einem Amin der allgemeinen Formel

$R_2R_3NH$ ,(V)

in der
$R_2$ und $R_3$ wie eingangs definiert sind.

Die Umsetzung wird vorzugsweise in einem Lösungsmittel wie Ethanol, Isopropanol, Tetrahydrofuran, Dioxan oder Benzol oder in einem Überschuß des eingesetzten Amins der allgemeinen Formel V gegebenenfalls in einem Druckgefäß und gegebenenfalls in Gegenwart einer Base wie Natriumcarbonat, Kaliumcarbonat, Triethylamin oder Pyridin bei Temperaturen zwischen 0 und 125°C, vorzugsweise bei Temperaturen zwischen 50 und 100°C, durchgeführt.

Bedeutet Y" in einer Verbindung der allgemeinen Formel IV eine Alkylthio- oder Phenylthiogruppe, so erhält man zweckmäßigerweise die entsprechende Sulfinylverbindung durch vorhergehende Oxidation mit einem Oxidationsmittel oder
bedeutet Y" in einer Verbindung der allgemeinen Formel IV eine Alkylthio-, Alkylsulfinyl-, Phenylthio- oder Phenylsulfinylgruppe, so erhält man zweckmäßigerweise die entsprechende Sulfonylverbindung durch vorhergehende Oxidation mit einem Oxidationsmittel.

Die vorhergehende Oxidation wird mit einem Oxidationsmittel wie einer Persäure, z. B. mit 3-Chlorperbenzoesäure, in einem Lösungsmittel wie Methylenchlorid, Ethanol, Isopropanol, Tetrahydrofuran, Dioxan oder Benzol bei Temperaturen zwischen 0 und 50°C, vorzugsweise bei Raumtemperatur, durchgeführt.

c) Zur Herstellung von Verbindungen der allgemeinen Formel I, in der R eine Alkylgruppe mit 1 bis 5 Kohlenstoffatomen darstellt:
Umsetzung einer Verbindung der allgemeinen Formel

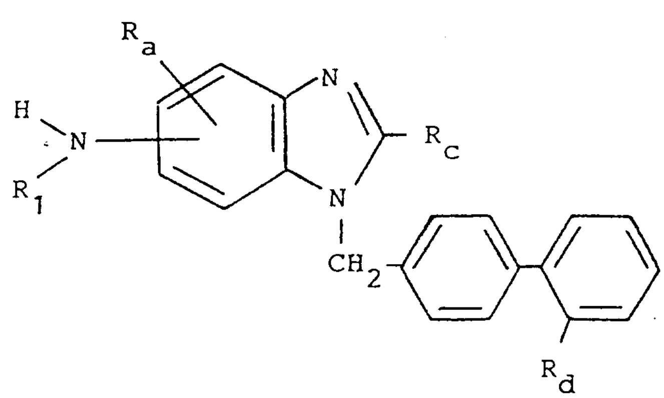

,(II)

in der
$R_a$, $R_c$, $R_d$ und $R_1$ wie eingangs definiert sind, mit einer Verbindung der allgemeinen Formel

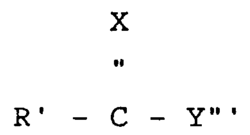

,(VI)

in der
X wie eingangs definiert ist,
R' eine Alkylgruppe mit 1 bis 5 Kohlenstoffatomen und
Y''' eine Alkylthiogruppe mit 1 bis 3 Kohlenstoffatomen darstellt.

Die Umsetzung wird vorzugsweise in einem Lösungsmittel wie Methanol, Äthanol, Isopropanol, Dioxan, Tetrahydrofuran oder Chloroform gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50 °C, vorzugsweise jedoch bei Raumtemperatur, durchgeführt.

d) Zur Herstellung einer Verbindung der allgemeinen Formel I, in der $R_d$ eine Carboxygruppe darstellt:
Überführung einer Verbindung der allgemeinen Formel

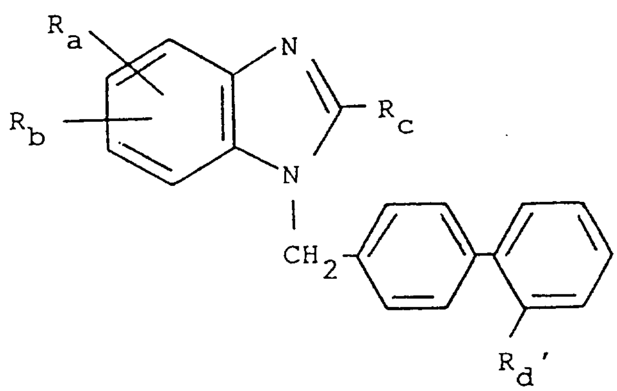

,(VII)

in der
$R_a$ bis $R_c$ wie eingangs definiert sind und
$R_d$' eine mittels Hydrolyse, Thermolyse oder Hydrogenolyse in eine Carboxygruppe überführbare Gruppe darstellt.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure oder besonders vorteilhaft in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Bedeutet $R_d$' in einer Verbindung der allgemeinen Formel VII eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50 °C in die Carboxygruppe übergeführt werden.

Bedeutet $R_d$' in einer Verbindung der allgemeinen Formel VII beispielsweise die tert. Butyloxycarbonylgruppe, so kann die tert. Butylgruppe auch thermisch gegebenenfalls in einem inerten Lösungsmittel

wie Methylenchlorid, Chloroform, Benzol, Toluol, Tetrahydrofuran oder Dioxan und vorzugsweise in Gegenwart einer katalytischen Menge einer Säure wie Trifluoressigsäure, p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 ° C und 100 ° C, abgespalten werden.

Bedeutet $R_d$' in einer Verbindung der allgemeinen Formel VII beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50 ° C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

e) Zur Herstellung einer Verbindung der allgemeinen Formel I, in der $R_d$ eine 1H-Tetrazolylgruppe darstellt:

Abspaltung eines Schutzrestes von einer Verbindung der allgemeinen Formel

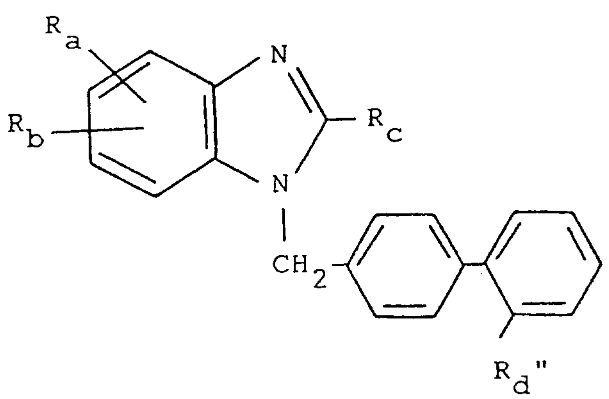

,(VIII)

in der

$R_a$ bis $R_c$ wie eingangs definiert sind und

$R_d$'' eine in 1- oder 2-Stellung durch einen Schutzrest geschützte 1H-Tetrazolyl- oder 2H-Tetrazolylgruppe darstellt.

Als Schutzrest kommt beispielsweise die Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100 ° C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150 ° C, vorzugsweise bei Temperaturen zwischen 120 und 140 ° C.

f) Zur Herstellung von Verbindungen der allgemeinen Formel I, in der $R_b$ eine $R_1$NH-Gruppe darstellt, in der das Wasserstoffatom durch eine der eingangs erwähnten 3-Nitro-pyrrol-2-yl-Reste ersetzt ist:

Cyclisierung einer gegebenenfalls im Reaktionsgemisch gebildeten Verbindung der allgemeinen Formel

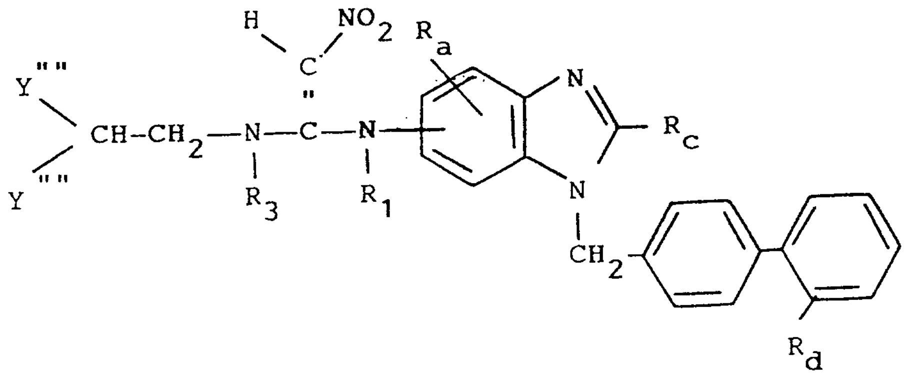

,(IX)

in der

$R_1$, $R_3$, $R_a$, $R_c$ und $R_d$ wie eingangs definiert sind und

die Reste Y'''', die gleich oder verschieden sein können, jeweils eine Alkoxygruppe mit 1 bis 3 Kohlenstoffatomen darstellen.

Die Cyclisierung wird vorzugsweise in einem Lösungsmittel wie Methanol, Ethanol, Isopropanol, Tetrahydrofuran, Dioxan oder Wasser oder in deren wässrigen Gemischen in Gegenwart einer Säure wie Salzsäure oder Schwefelsäure bei Temperaturen zwischen 0 und 125 °C, vorzugsweise bei Temperaturen zwischen 50 und 100 °C, durchgeführt. Die hierzu erforderliche Verbindung der allgemeinen Formel IX erhält man zweckmäßigerweise durch Umsetzung einer entsprechenden Verbindung der allgemeinen Formel IV mit einem 2-Amino-acetaldehyddiacetal in einem Lösungsmittel wie Ethanol, Isopropanol, Tetrahydrofuran, Dioxan oder Benzol oder in einem Überschuß des eingesetzten 2-Aminoacetaldehyddiacetals gegebenenfalls in Gegenwart einer Base wie Natriumcarbonat, Kaliumcarbonat, Triethylamin oder Pyridin bei Temperaturen zwischen 0 und 125 °C, vorzugsweise bei Temperaturen zwischen 50 und 100 °C, durchgeführt.

g) Zur Herstellung von Verbindungen der allgemeinen Formel I, in der $R_b$ eine $R_1$NH-Gruppe darstellt, in der das Wasserstoffatom durch eine der eingangs erwähnten 3,4-Dioxo-1-cyclobuten-1-yl-Reste ersetzt ist:

Umsetzung einer Verbindung der allgemeinen Formel

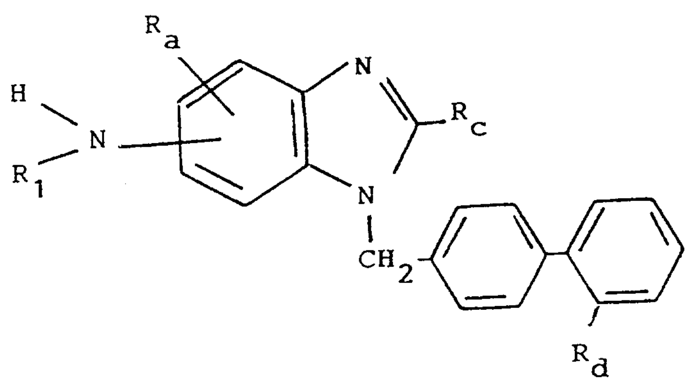

,(II)

in der

$R_a$, $R_c$, $R_d$ und $R_1$ wie eingangs definiert sind, mit einem 3,4-Dioxo-1,3-dialkoxy-1-cyclobuten mit jeweils 1 bis 3 Kohlenstoffatomen im Alkoxyteil und

zur Herstellung einer entsprechenden 2-Aminoverbindung anschließende Umsetzung einer der so erhaltenen 2-Alkoxyverbindung mit einem Amin der allgemeinen Formel

$R_2R_3NH$ ,(V)

in der

$R_2$ und $R_3$ wie eingangs definiert sind.

Die Umsetzung wird vorzugsweise in einem Lösungsmittel wie Methanol, Äthanol, Isopropanol, Dioxan, Tetrahydrofuran oder Chloroform gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50 °C, vorzugsweise jedoch bei Raumtemperatur, durchgeführt.

Die anschließende Umsetzung einer so erhaltenen 2-Alkoxyverbindung mit einem Amin der allgemeinen Formel V wird vorzugsweise in einem Lösungsmittel wie Ethanol, Isopropanol, Tetrahydrofuran, Dioxan oder Benzol oder in einem Überschuß des eingesetzten Amins der allgemeinen Formel V gegebenenfalls in einem Druckgefäß und gegebenenfalls in Gegenwart einer Base wie Natriumcarbonat, Kaliumcarbonat, Triethylamin oder Pyridin bei Temperaturen zwischen 0 und 125 °C, vorzugsweise bei Temperaturen zwischen 50 und 100 °C, durchgeführt.

h) Zur Herstellung einer Verbindung der allgemeinen Formel I, in der $R_d$ eine 1H-Tetrazolylgruppe darstellt:

Umsetzung einer Verbindung der allgemeinen Formel

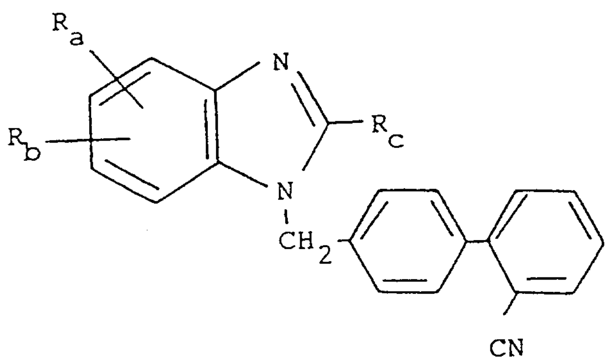

, (X)

in der

$R_a$ bis $R_c$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150 °C, vorzugsweise bei 125 °C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Ein erfindungsgemäß so erhaltenes Isomerengemisch einer Verbindung der allgemeinen Formel I kann gewünschtenfalls vorzugsweise chromatographisch unter Verwendung eines Trägers wie Kieselgel oder

Aluminiumoxid getrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis X sind teilweise literaturbekannt oder man erhält diese nach literaturbekannten Verfahren, wie diese beispielsweise in der EP-A-0,253,310, EP-A-0,291,969, EP-A-0,392,317, EP-A-0,468,470 und EP-A-0,502,314 beschrieben werden.

So erhält man beispielsweise eine Verbindung der allgemeinen Formel II durch Abspaltung eines Schutzrestes von einer entsprechend substituierten Aminoverbindung, welche man ihrerseits durch Acylierung eines entsprechenden o-Phenylendiamins und anschließender Cyclisierung oder durch Acylierung einer entsprechenden o-Amino-nitroverbindung, anschließender Reduktion der Nitrogruppe und Cyclisierung, wobei ein so gegebenenfalls erhaltenes NH-Benzimidazol mittels Alkylierung mit einem entsprechenden Biphenylderivat in eine in 1-Stellung entsprechend substituierte Verbindung übergeführt werden kann, und gegebenenfalls anschließende Abspaltung eines verwendeten Schutzrestes.

Eine als Ausgangsstoff verwendete Verbindung der allgemeinen Formel IV erhält man durch Umsetzung einer Verbindung der allgemeinen Formel II mit einer entsprechenden Verbindung der allgemeinen Formel III,

eine Verbindung der allgemeinen Formel VII und VIII durch Umsetzung eines entsprechenden 1H-Benzimidazols mit einem entsprechenden Biphenylderivat.

Enthält der Rest $R_b$ in einer Ausgangsverbindung der allgemeinen Formel VII oder VIII einen der eingangs erwähnten 3-Nitro-pyrrol-Reste, so erhält man diese durch Umsetzung einer entsprechenden 1-Alkylmercapto-2-nitro-ethenylenamino-Verbindung mit einem entsprechenden 2-Amino-acetaldehydacetal und anschließende Cyclisierung in Gegenwart einer Säure (siehe auch J. Med. Chem. <u>31</u>, 669 (1988)).

Enthält der Rest $R_b$ in einer Ausgangsverbindung der allgemeinen Formel VII oder VIII eine der eingangs erwähnten 3,4-Dioxo-1-cyclobuten-Reste, so erhält man diese durch Umsetzung einer entsprechenden Aminoverbindung mit einem 1,2-Dialkoxy-3,4-dioxo-1-cyclobuten und anschließende Umsetzung mit einem entsprechenden Amin (siehe auch J. Med. Chem. <u>35</u>, 4720 (1992)).

Eine Ausgangsverbindung der allgemeinen Formel IX erhält man durch Umsetzung eines entsprechenden 1-Alkylmercapto-2-nitroethenylenamins der allgemeinen Formel I mit einem 2-Aminoacetaldehyddiacetal.

Eine als Ausgangsstoff verwendete Verbindung der allgemeinen Formel VI erhält man durch Umsetzung einer entsprechend substituierten Dialkylthioverbindung mit einem entsprechenden Alkylmagnesiumhalogenid.

Die bei der Herstellung der Ausgangsverbindungen gegebenenfalls erforderliche Monoalkylierung von Aminen wird in J. Chem. Soc. Chem. Com. <u>1984</u>, 1335 beschrieben.

Die neuen Verbindungen der allgemeinen Formel I, in denen $R_d$ eine in vivo in eine Carboxygruppe überführbare Gruppe, eine Carboxy- oder eine 1H-Tetrazolylgruppe darstellt, und deren physiologisch verträgliche Salze weisen wertvolle pharmakologische Eigenschaften auf. Sie stellen Angiotensin-Antagonisten, insbesondere Angiotensin-II-Antagonisten, dar. Die übrigen Verbindungen der allgemeinen Formel I stellen wertvolle Zwischenprodukte zur Herstellung der vorstehend erwähnten Verbindung dar.

Beispielsweise wurden die Verbindungen

A = 4'-[[2-n-Propyl-4-methyl-6-(2-cyano-3-methyl-guanidino)-1H-benzimidazol-1-yl)-methyl]-biphenyl-2-carbonsäure,

B = 4'-[[2-n-Propyl-4-methyl-6-(2-cyano-3,3-dimethyl-guanidino)-1H-benzimidazol-1-yl]-methyl]-2-(1H-tetrazol-5-yl)-biphenyl,

C = 4'-[[2-n-Propyl-4-methyl-6-(2,2-dicyano-1-dimethylaminoethenylenamino)-1H-benzimidazol-1-yl]-methyl]-biphenyl-2-carbonsäure,

D = 4'-[[2-n-Propyl-4-methyl-6-(1-dimethylamino-2-nitroethenylenamino)-1H-benzimidazol-1-yl]-methyl]-biphenyl-2-carbonsäure,
E = 4'-[[2-n-Propyl-4-methyl-6-(3-methyl-2-amidosulfonylguanidino)-1H-benzimidazol-1-yl]-methyl]-biphenyl-2-carbonsäure,
F = 4'-[[2-n-Propyl-4-methyl-6-(1,1-dimethylamino-2-nitroethylenamino)-1H-benzimidazol-1-yl]-methyl]-2-(1H-tetrazol-5-yl)-biphenyl,
G = 4'-[[2-Ethyl-4-methyl-6-[(2-dimethylamino-3,4-dioxo-1-cyclobuten-1-yl)-amino]-1H-benzimidazol-1-yl]-methyl]-biphenyl-2-carbonsäure und
H = 4'-[[2-Ethyl-4-methyl-6-(3,3-dimethyl-2-methylsulfonylguanidino)-1H-benzimidazol-1-yl]-methyl]-2-(1H-tetrazol-5-yl)-biphenyl

auf ihre biologischen Wirkungen wie folgt untersucht:

Methodenbeschreibung Anqiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37°C mit 50 pM [$^{125}$I]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis H zeigen in dem beschriebenen Test folgende $IC_{50}$-Werte:

| Substanz | $IC_{50}$ [nM] |
|---|---|
| A | 11 |
| B | 3 |
| C | 64 |
| D | 13 |
| E | 56 |
| F | 2 |
| G | 17 |
| H | 4 |

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'-schen Krankheit, des Parkinson-Syndroms, der Bulimie, sowie von Störungen kognitiver Funktionen.

Die zur Erzielung einer entsprechenden Wirkung am Erwachsenen erforderliche Dosierung beträgt zweckmäßigerweise bei intravenöser Gabe 0,5 bis 100 mg, vorzugsweise 1 bis 70 mg, und bei oraler Gabe 0,1 bis 200 mg, vorzugsweise 1 bis 100 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, ACE-Hemmer, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon,

Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

Beispiel 1

4'-[[2-n-Propyl-4-chlor-6-(2-cyano-3-methyl-guanidino)-1H-benzimidazol-1-yl]-methyl]-biphenyl-2-carbonsäure

a) 4'-[(2-n-Propyl-4-chlor-6-phthalimido-1H-benzimidazol-1-yl)-methyl]-biphenyl-2-carbonsäure-methylester

3,27 g (10,0 mMol) 2-n-Propyl-4-chlor-6-phthalimido-1H-benzimidazol werden in 50 ml Dimethylsulfoxid gelöst, portionsweise mit 1,23 g (11,0 mMol) Kalium-tert.butylat versetzt und 30 Minuten bei Raumtemperatur gerührt. Danach werden 3,38 g (11,0 mMol) 4'-Brommethyl-biphenyl-2-carbonsäuremethylester portionsweise zugegeben. Anschließend wird das Reaktionsgemisch 3 Stunden bei Raumtemperatur gerührt, in Eiswasser eingerührt und 3 x mit Essigester extrahiert. Die vereinigten organischen Phasen werden mit Kochsalzlösung gewaschen und über Magnesiumsulfat getrocknet. Anschließend wird das Solvens im Vakuum entfernt und der Rückstand an Kieselgel (Korngröße: 0,032-0,063 mm) chromatographiert, wobei als Elutionsmittel anfangs Petrolether, später Gemische von Petrolether und Essigester steigender Polarität (9:1, 4:1 und 7:3) verwendet werden. Die einheitlichen Fraktionen werden vereinigt und eingedampft.
Ausbeute: 2,15 g (38 % der Theorie),
Schmelzpunkt: sintert ab 118 °C

b) 4'-[[2-n-Propyl-4-chlor-6-amino-1H-benzimidazol-1-yl)-methyl]-biphenyl-2-carbonsäure-methylester

2,05 g 4'-[(2-n-Propyl-4-chlor-6-phthalimido-1H-benzimidazol-1-yl)-methyl]-biphenyl-2-carbonsäure-methylester werden in 125 ml Ethanol gelöst und mit 5 ml einer 40%igen wäßrigen Methylaminlösung versetzt. Das Reaktionsgemisch wird 3 Stunden bei Raumtemperatur gerührt und anschließend in Kochsalzlösung eingerührt. Nach 3-facher Extraktion mit Essigester werden die vereinigten organischen Phasen über Natriumsulfat getrocknet und eingedampft. Der Rückstand wird 24 Stunden mit Petrolether/Ether (1:4) gerührt, der dabei gebildete Feststoff wird abgesaugt und getrocknet.
Ausbeute: 745 mg (50 % der Theorie),
Schmelzpunkt: 120-121 °C

c) 4'-[[2-n-Propyl-4-chlor-6-(2-cyano-3-methyl-guanidino)-1H-benzimidazol-1-yl)-methyl]-biphenyl-2-carbonsäure-methylester

0,7 g (1,6 mMol) 4'-[[2-n-Propyl-4-chlor-6-amino-1H-benzimidazol-1-yl)-methyl]-biphenyl-2-carbonsäure-methylester und 395 mg (1,6 mMol) Cyancarbamidsäure-diphenylester werden in 75 ml Isopropanol gelöst und 18 Stunden bei Raumtemperatur gerührt. Danach wird von der entstandenen Trübung abfiltriert. Das Filtrat wird in einem Druckgefäß mit ca. 1 ml gasförmigem Methylamin versetzt und 4 Stunden auf 100 °C erhitzt. Nach Abkühlung wird das Reaktionsgemisch eingedampft und der Rückstand mit 100 ml Kochsalzlösung versetzt. Nach 3-facher Extraktion mit Essigester werden die vereinigten organischen Phasen über Natriumsulfat getrocknet. Das Rohprodukt wird an Kieselgel (Korngröße: 0,032-0,063 mm) chromatographiert, wobei als Elutionsmittel anfangs Methylenchlorid, später Methylenchlorid/Ethanol (50:1, 25:1 und 9:1) verwendet wird. Die einheitlichen Fraktionen werden vereinigt und eingedampft.
Ausbeute: 205 mg (21 % der Theorie),

Schmelzpunkt: > 250 °C (Zers.)

d) 4'-[[2-n-Propyl-4-chlor-6-(2-cyano-3-methyl-guanidino)-1H-benzimidazol-1-yl)-methyl]-biphenyl-2-carbonsäure

175 mg (0,34 mMol) 4'-[(2-n-Propyl-4-chlor-6-(2-cyano-3-methyl-guanidino)-1H-benzimidazol-1-yl)-methyl]-biphenyl-2-carbonsäure-methylester werden in 20 ml Ethanol gelöst, mit 2 ml 2N Natronlauge versetzt und 16 Stunden bei Raumtemperatur gerührt. Nach Abdampfen des Solvens im Vakuum wird der viskose Rückstand in 50 ml Bicarbonatlösung aufgenommen, über Aktivkohle filtriert und bei 0 °C mit 2N Essigsäure versetzt. Der dabei gebildete Niederschlag wird abgesaugt, mit Wasser gewaschen und getrocknet.
Ausbeute: 95 mg (56 % der Theorie),
Schmelzpunkt: 251-254 °C
$C_{27}H_{25}ClN_6O_2$ (500,99)

| | | | | | | | | |
|---|---|---|---|---|---|---|---|---|
| Ber.: | C | 64,50 | H | 5,00 | N | 16,80 | Cl | 7,10 |
| Gef.: | | 64,33 | | 5,13 | | 16,77 | | 7,21 |

Beispiel 2

4'-[[2-n-Propyl-4-methyl-6-(2-cyano-3-methyl-guanidino)-1H-benzimidazol-1-yl)-methyl]-biphenyl-2-carbonsäure

a) 4'-[(2-n-Propyl-4-methyl-6-phthalimido-1H-benzimidazol-1-yl)-methyl]-biphenyl-2-carbonsäure-methylester

Hergestellt analog Beispiel 1a aus 2-n-Propyl-4-methyl-6-phthalimido-1H-benzimidazol und 4'-Brommethyl-biphenyl-2-carbonsäure-methylester.
Ausbeute: 35 % der Theorie,
Schmelzpunkt: 176-177 °C

b) 4'-[(2-n-Propyl-4-methyl-6-amino-1H-benzimidazol-1-yl)-methyl]-biphenyl-2-carbonsäure-methylester

Hergestellt analog Beispiel 1b aus 4'-[(2-n-Propyl-4-methyl-6-phthalimido-1H-benzimidazol-1-yl)-methyl]-biphenyl-2-carbonsäure-methylester und Methylamin.
Ausbeute: 78 % der Theorie,
$R_f$-Wert: 0,40 (Kieselgel; Essigester/Petrolether = 4:1)

c) 4'-[[2-n-Propyl-4-methyl-6-(2-cyano-3-methyl-guanidino)-1H-benzimidazol-1-yl]-methyl]-biphenyl-2-carbonsäure-methylester

Hergestellt analog Beispiel 1c aus 4'-[(2-n-Propyl-4-methyl-6-amino-1H-benzimidazol-1-yl)-methyl]-biphenyl-2-carbonsäure-methylester, Cyancarbamidsäure-diphenylester und Methylamin.
Ausbeute: 44 % der Theorie,
Schmelzpunkt: 117-119 °C

d) 4'-[[2-n-Propyl-4-methyl-6-(2-cyano-3-methyl-guanidino)-1H-benzimidazol-1-yl]-methyl]-biphenyl-2-carbonsäure

Hergestellt analog Beispiel 1d aus 4'-[[2-n-Propyl-4-methyl-6-(2-cyano-3-methyl-guanidino)-1H-benzimidazol-1-yl)-methyl]-biphenyl-2-carbonsäure-methylester und 2N Natronlauge in Ethanol.
Ausbeute: 40 % der Theorie,
Schmelzpunkt: 247-249 °C
$C_{28}H_{28}N_6O_2$ (480,57)
Massenspektrum: $M^+$ = 480

Beispiel 3

4'-[[2-n-Propyl-4-methyl-6-(2-cyano-3,3-dimethyl-guanidino)-1H-benzimidazol-1-yl]-methyl]-biphenyl-2-carbonsäure

a) 4'-[[2-n-Propyl-4-methyl-6-(2-cyano-3,3-dimethyl-guanidino)-1H-benzimidazol-1-yl]-methyl]-biphenyl-2-carbonsäure-methylester

Hergestellt analog Beispiel 1c aus 4'-[[2-n-Propyl-4-methyl-6-amino-1H-benzimidazol-1-yl)-methyl]-biphenyl-2-carbonsäure-methylester, Cyancarbamidsäure-diphenylester und Dimethylamin.
Ausbeute: 34 % der Theorie,
$R_f$-Wert: 0,60 (Kieselgel; Methylenchlorid/Ethanol = 9:1)

b) 4'-[[2-n-Propyl-4-methyl-6-(2-cyano-3,3-dimethyl-guanidino)-1H-benzimidazol-1-yl]-methyl]-biphenyl-2-carbonsäure

Hergestellt analog Beispiel 1d aus 4'-[[2-n-Propyl-4-methyl-6-(2-cyano-3,3-dimethyl-guanidino)-1H-benzimidazol-1-yl]-methyl]-biphenyl-2-carbonsäuremethylester und 2N Natronlauge in Ethanol.
Ausbeute: 9,5 % der Theorie,
Schmelzpunkt: sintert ab 180 ° C
$C_{29}H_{30}N_6O_2$ (494,60)
Massenspektrum: $M^+$ = 494

Beispiel 4

4'-[[2-n-Propyl-4-chlor-6-(2-cyano-3,3-dimethyl-guanidino)-1H-benzimidazol-1-yl]-methyl]-2-(1H-tetrazol-5-yl)-biphenyl

a) 4'-[(2-n-Propyl-4-chlor-6-phthalimido-1H-benzimidazol-1-yl)-methyl]-2-(1-triphenylmethyl-tetrazol-5-yl)-biphenyl

Hergestellt analog Beispiel 1a aus 2-n-Propyl-4-chlor-6-phthalimido-1H-benzimidazol und 4'-Brommethyl-2-(1-triphenylmethyl-tetrazol-5-yl)-biphenyl.
Ausbeute: 35 % der Theorie,
$R_f$-Wert: 0,55 (Kieselgel; Methylenchlorid/Ethanol = 19:1)

b) 4'-[(2-n-Propyl-4-chlor-6-amino-1H-benzimidazol-1-yl)-methyl]-2-(1-triphenylmethyl-tetrazol-5-yl)-biphenyl

Hergestellt analog Beispiel 1b aus 4'-[(2-n-Propyl-4-chlor-6-phthalimido-1H-benzimidazol-1-yl)-methyl]-2-(1-triphenylmethyl-tetrazol-5-yl)-biphenyl und Methylamin.
Ausbeute: 72 % der Theorie,
Schmelzpunkt: 209-210 ° C

c) 4'-[[2-n-Propyl-4-chlor-6-(2-cyano-3,3-dimethyl-guanidino)-1H-benzimidazol-1-yl]-methyl]-2-(1-triphenylmethyltetrazol-5-yl)-biphenyl

Hergestellt analog Beispiel 1c aus 4'-[(2-n-Propyl-4-chlor-6-amino-1H-benzimidazol-1-yl)-methyl]-2-(1-triphenylmethyltetrazol-5-yl)-biphenyl, Cyancarbamidsäure-diphenylester und Dimethylamin.
Ausbeute: 50 % der Theorie,
Schmelzpunkt: sintert ab 134 ° C
Daneben entstehen 18 % 4'-[[2-n-Propyl-4-chlor-6-(2-cyano-3,3-dimethyl-guanidino)-1H-benzimidazol-1-yl]-methyl]-2-(1H-tetrazol-5-yl)-biphenyl

d) 4'-[[2-n-Propyl-4-chlor-6-(2-cyano-3,3-dimethyl-guanidino)-1H-benzimidazol-1-yl)-methyl]-2-(1H-tetrazol-5-yl)-biphenyl

225 mg (0,29 mMol) 4'-[[2-n-Propyl-4-chlor-6-(2-cyano-3,3-dimethyl-guanidino)-1H-benzimidazol-1-yl)-methyl]-2-(1-triphenylmethyl-tetrazol-5-yl)-biphenyl werden in 10 ml absolutem Ethanol gelöst, mit 2,5 ml

methanolischer Salzsäure versetzt und 90 Minuten bei Raumtemperatur gerührt. Anschließend wird das Solvens im Vakuum entfernt und der Rückstand in Methylenchlorid/Ethanol (4:1) aufgenommen und mit methanolischem Ammoniak auf pH 8 eingestellt. Nach Zusatz von 5 g Kieselgel wird zur Trockne eingedampft. Der Rückstand wird an Kieselgel (Korngröße: 0,032-0,063 mm) chromatographiert, wobei als Elutionsmittel anfangs Methylenchlorid, später Methylenchlorid/Ethanol (50:1, 25:1, 19:1 und 9:1) verwendet wird. Die einheitlichen Fraktionen werden vereinigt und eingedampft.
Ausbeute: 15 mg (10 % der Theorie),
Schmelzpunkt: 204-206 °C
$C_{28}H_{27}ClN_{10}$ (539,05)
Massenspektrum: $(M+H)^+$ = 539/541 (Cl)

Beispiel 5

4'-[[2-n-Propyl-4-chlor-6-(2-cyano-3-methyl-guanidino)-1H-benzimidazol-1-yl)-methyl]-2-(1H-tetrazol-5-yl)-biphenyl

a) 4'-[[2-n-Propyl-4-chlor-6-(2-cyano-3-methyl-guanidino)-1H-benzimidazol-1-yl)-methyl]-2-(1-triphenylmethyl-tetrazol-5-yl)-biphenyl

Hergestellt analog Beispiel 1c aus 4'-[(2-n-Propyl-4-chlor-6-amino-1H-benzimidazol-1-yl)-methyl]-2-(1-triphenylmethyl tetrazol-5-yl)-biphenyl, Cyancarbamidsäure-diphenylester und Methylamin.
Ausbeute: 32 % der Theorie,
Schmelzpunkt: 222-224 °C

b) 4'-[[2-n-Propyl-4-chlor-6-(2-cyano-3-methyl-guanidino)-1H-benzimidazol-1-yl)-methyl]-2-(1H-tetrazol-5-yl)-biphenyl

Hergestellt analog Beispiel 4d aus 4'-[(2-n-Propyl-4-chlor-6-(2-cyano-3-methyl-guanidino)-1H-benzimidazol-1-yl]-methyl]-2-(1-triphenylmethy-tetrazol-5-yl)-biphenyl und methanolischer Salzsäure.
Ausbeute: 11 % der Theorie,
Schmelzpunkt: >250 °C
$C_{27}H_{25}ClN_{10}$ (525,02)
Massenspektrum: $(M+H)^+$ = 525/527 (Cl)

Beispiel 6

4'-[[2-n-Propyl-4-methyl-6-(2-cyano-3-cyclohexyl-guanidino)-1H-benzimidazol-1-yl]-methyl]-2-(1H-tetrazol-5-yl)-biphenyl

a) 4'-[(2-n-Propyl-4-methyl-6-phthalimido-1H-benzimidazol-1-yl]-methyl]-2-cyano-biphenyl

Hergestellt analog Beispiel 1a aus 2-n-Propyl-4-methyl-6-phthalimido-1H-benzimidazol und 4'-Brommethyl-2-cyano-biphenyl.
Ausbeute: 49 % der Theorie,
Schmelzpunkt: 243-244 °C

b) 4'-[[2-n-Propyl-4-methyl-6-amino-1H-benzimidazol-1-yl]-methyl]-2-(1H-tetrazol-5-yl)-biphenyl

4,75 g (12,5 mMol) 4'-[(2-n-Propyl-4-methyl-6-phthalimido-1H-benzimidazol-1-yl]-methyl]-2-cyano-biphenyl werden in 200 ml absolutem Toluol gelöst und mit 20,8 g (62,5 mMol) Tributylzinnazid versetzt. Das Reaktionsgemisch wird 5 Tage unter Rühren zum Rückfluß erhitzt. Anschließend wird das Solvens abdestilliert, der Rückstand in Kochsalzlösung aufgenommen und 3 x mit je 100 ml Essigester extrahiert. Die vereinigten organischen Phasen werden über Natriumsulfat getrocknet und eingedampft. Der Rückstand wird an Kieselgel (Korngröße: 0,032-0,063 mm) chromatographiert, wobei als Elutionsmittel anfangs Methylenchlorid, später Methylenchlorid/Ethanol (50:1, 25:1 und 9:1) verwendet wird. Die einheitlichen Fraktionen werden vereinigt und eingedampft. Der Rückstand wird mit Ether/Petrolether (1:1) verrieben und abgesaugt.
Ausbeute: 4,05 g (77 % der Theorie),
Schmelzpunkt: sintert ab 177 °C

c) 4'-[[2-n-Propyl-4-methyl-6-(2-phenoxy-3-cyano-isoureido)-1H-benzimidazol-1-yl]-2-(1H-tetrazol-5-yl)-biphenyl

1,70 g (4,0 mMol) 4'-[[2-n-Propyl-4-methyl-6-amino-1H-benzimidazol-1-yl]-methyl]-2-(1H-tetrazol-5-yl)-biphenyl werden in 150 ml Isopropanol aufgenommen und mit 30 ml Methylenchlorid und 3,1 g (13 mMol) Cyancarbamidsäure-diphenylester versetzt. Die Reaktionslösung wird 3 Tage bei Raumtemperatur gerührt und anschließend eingedampft. Der Rückstand wird an Kieselgel (Korngröße: 0,032-0,063 mm) chromatographiert, wobei als Elutionsmittel anfangs Methylenchlorid, später Methylenchlorid/Ethanol (50:1, 25,:1 und 9:1) verwendet wird. Die einheitlichen Fraktionen werden vereinigt und eingedampft. Der Rückstand wird mit Ether/Petrolether (1:1) verrieben und abgesaugt.
Ausbeute: 905 mg (40 % der Theorie),
Schmelzpunkt: >250 °C

d) 4'-[[2-n-Propyl-4-methyl-6-(2-cyano-3-cyclohexyl-guanidino)-1H-benzimidazol-1-yl]-methyl]-2-(1H-tetrazol-5-yl) biphenyl

Hergestellt analog Beispiel 1c aus 4'-[[2-n-Propyl-4-methyl-6-(2-phenoxy-3-cyano-isoureido)-1H-benzimidazol-1-yl]-methyl]-2-(1H-tetrazol-5-yl)-biphenyl und Cyclohexylamin in siedendem Isopropanol.
Ausbeute: 44 % der Theorie,
Schmelzpunkt: sintert ab 188 °C
$C_{33}H_{36}N_{10}$ (572,72)
Massenspektrum: $(M+H)^+$ = 573

Beispiel 7

4'-[[2-n-Propyl-4-methyl-6-(2-cyano-3,3-dimethyl-guanidino)-1H-benzimidazol-1-yl]-methyl]-2-(1H-tetrazol-5-yl)-biphenyl

Hergestellt analog Beispiel 1c aus 4'-[[2-n-Propyl-4-methyl-6-(2-phenoxy-3-cyano-isoureido)-1H-benzimidazol-1-yl]-methyl]-2-(1H-tetrazol-5-yl)-biphenyl und Dimethylamin in siedendem Isopropanol.
Ausbeute: 24 % der Theorie,
Schmelzpunkt: sintert ab 202 °C
$C_{29}H_{30}N_{10}$ (518,63)
Massenspektrum: $M^+$ = 518

Beispiel 8

4'-[[2-n-Propyl-4-methyl-6-(2-cyano-3-isopropyl-guanidino)-1H-benzimidazol-1-yl]-methyl]-2-(1H-tetrazol-5-yl)-biphenyl

Hergestellt analog Beispiel 1c aus 4'-[[2-n-Propyl-4-methyl-6-(2-phenoxy-3-cyano-isoureido)-1H-benzimidazol-1-yl]-methyl)-2-(1H-tetrazol-5-yl)-biphenyl und Isopropylamin in siedendem Isopropanol.
Ausbeute: 39 % der Theorie,
Schmelzpunkt: sintert ab 198 °C
$C_{30}H_{32}N_{10}$ (532,66)
Massenspektrum: $M^+$ = 532

Beispiel 9

4'-[[2-n-Propyl-4-methyl-6-(2-cyano-3,3-diisopropyl-guanidino)-1H-benzimidazol-1-yl]-methyl]-2-(1H-tetrazol-5-yl)-biphenyl

Hergestellt analog Beispiel 1c aus 4'-[[2-n-Propyl-4-methyl-6-(2-phenoxy-3-cyano-isoureido)-1H-benzimidazol-1-yl]-methyl]-2-(1H-tetrazol-5-yl)-biphenyl und Diisopropylamin in siedendem Isopropanol.
Ausbeute: 19 % der Theorie,
Schmelzpunkt: sintert ab 185 °C
$C_{33}H_{38}N_{10}$ (574,74)
Massenspektrum: $(M+H)^+$ = 575

Beispiel 10

4'-[[2-n-Propyl-4-methyl-6-(2,2-dicyano-1-pyrrolidino-ethenylenamino)-1H-benzimidazol-1-yl]-methyl]-biphenyl-2-carbonsäure

a) 4'-[(2-n-Propyl-4-methyl-6-phthalimido-1H-benzimidazol-1-yl]-methyl]-biphenyl-2-carbonsäure-tert.butylester

Hergestellt analog Beispiel 1a aus 2-n-Propyl-4-methyl-6-phthalimido-1H-benzimidazol und 4'-Brommethyl-biphenyl-2-carbonsäure-tert.butylester.
Ausbeute: 68 % der Theorie,
Schmelzpunkt: 153-154 °C

b) 4'-[(2-n-Propyl-4-methyl-6-amino-1H-benzimidazol-1-yl)-methyl]-biphenyl-2-carbonsäure-tert.butylester

Hergestellt analog Beispiel 1b aus 4'-[(2-n-Propyl-4-methyl-6-phthalimido-1H-benzimidazol-1-yl)-methyl]-biphenyl-2-carbonsäure-tert.butylester und Methylamin.
Ausbeute: 94 % der Theorie,
Schmelzpunkt: 118-119 °C

c) 4'-[[2-n-Propyl-4-methyl-6-(2,2-dicyano-1-methylmercaptoethenylenamino)-1H-benzimidazol-1-yl)-methyl]-biphenyl-2-carbonsäure-tert.butylester

2,70 g (6,1 mMol) 4'-[[2-n-Propyl-4-methyl-6-amino-1H-benzimidazol-1-yl)-methyl]-biphenyl-2-carbonsäure-tert.butylester werden in 125 ml Isopropanol gelöst, mit 2,38 g (14 mMol) 2,2-Dicyano-1,1-dimethylmercapto-ethen versetzt und 4 Tage unter Rückfluß erhitzt. Danach wird das Solvens abgedampft, der Rückstand wird an Kieselgel (Korngröße: 0,032-0,063 mm) chromatographiert, wobei als Elutionsmittel anfangs Methylenchlorid, später Methylenchlorid/Ethanol (50:1 und 25:1) verwendet wird. Die einheitlichen Fraktionen werden vereinigt und eingedampft. Der Rückstand wird mit Ether verrieben und abgesaugt.
Ausbeute: 2,15 g (62 % der Theorie),
Schmelzpunkt: 186-188 °C

d) 4'-[[2-n-Propyl-4-methyl-6-(2,2-dicyano-1-pyrrolidinoethenylenamino)-1H-benzimidazol-1-yl]-methyl]-biphenyl-2-carbonsäure-tert.butylester

425 mg (0,75 mMol) 4'-[[2-n-Propyl-4-methyl-6-(2,2-dicyano-1-methylmercapto-ethenylenamino)-1H-benzimidazol-1-yl]-methyl]-biphenyl-2-carbonsäure-tert.butylester werden in 25 ml Methylenchlorid gelöst. Anschließend wird bei 0 °C unter Stickstoff eine Lösung von 380 mg (1,1 mMol) 3-Chlor-peroxybenzoesäure (50%ig) in 5 ml Methylenchlorid zugetropft. Die Lösung wird 5 Stunden bei Raumtemperatur gerührt, anschließend mit 1,17 g (16,5 mMol) Pyrrolidin versetzt und weitere 12 Stunden bei Raumtemperatur gerührt. Danach wird das Solvens abgedampft, der Rückstand wird an Kieselgel (Korngröße: 0,032-0,063 mm) chromatographiert, wobei als Elutionsmittel anfangs Methylenchlorid, später Methylenchlorid/Ethanol (50:1, 25:1 und 19:1) verwendet wird. Die einheitlichen Fraktionen werden vereinigt und eingedampft.
Ausbeute: 185 mg (41 % der Theorie),
$R_f$-Wert: 0,40 (Kieselgel; Methylenchlorid/Ethanol = 19:1)

e) 4'-[[2-n-Propyl-4-methyl-6-(2,2-dicyano-1-pyrrolidino-ethenylenamino)-1H-benzimidazol-1-yl]-methyl]-biphenyl-2-carbonsäure

185 mg (0,31 mMol) 4'-[[2-n-Propyl-4-methyl-6-(2,2-dicyano-1-pyrrolidino-ethenylenamino)-1H-benzimidazol-1-yl]-methyl]-biphenyl-2-carbonsäure-tert.butylester werden in 10 ml Methylenchlorid gelöst und mit 1,5 ml Trifluoressigsäure versetzt. Nach 3 Stunden bei Raumtemperatur wird die Lösung eingedampft, der Rückstand wird in 25 ml Natriumbicarbonatlösung aufgenommen und über Aktivkohle filtriert. Der nach Zugabe von 2N Essigsäure bei 5 °C gebildete Niederschlag wird abgesaugt und getrocknet.
Ausbeute: 37 mg (22 % der Theorie),
Schmelzpunkt: 188-190 °C
$C_{33}H_{32}N_6O_2$ (544,66)
Massenspektrum: $(M+H)^+ = 545$

Beispiel 11

4'-[[2-n-Propyl-4-methyl-6-(2,2-dicyano-1-methylamino-ethenylenamino)-1H-benzimidazol-1-yl]-methyl]-biphenyl-2-carbonsäure

a) 4'-[[2-n-Propyl-4-methyl-6-(2,2-dicyano-1-methylaminoethenylenamino)-1H-benzimidazol-1-yl]-methyl]-biphenyl-2-carbonsäure-tert.butylester

Hergestellt analog Beispiel 10d aus 4'-[(2-n-Propyl-4-methyl-6-(2,2-dicyano-1-methylmercapto-ethenylenamino)-1H-benzimidazol-1-yl]-methyl]-biphenyl-2-carbonsäure-tert.butylester, 3-Chlor-peroxybenzoesäure und Methylamin.
Ausbeute: 45 % der Theorie,
Schmelzpunkt: 240-242 ° C

b) 4'-[[2-n-Propyl-4-methyl-6-(2,2-dicyano-1-methylaminoethenylenamino)-1H-benzimidazol-1-yl]-methyl]-biphenyl-2-carbonsäure

Hergestellt analog Beispiel 10e aus 4'-[[2-n-Propyl-4-methyl-6-(2,2-dicyano-1-methylamino-ethenylenamino)-1H-benzimidazol-1-yl]-methyl]-biphenyl-2-carbonsäure-tert.butylester und Trifluoressigsäure.
Ausbeute: 43 % der Theorie,
Schmelzpunkt: sintert ab 178 ° C
$C_{30}H_{28}N_6O_2$ (504,60)
Massenspektrum: $M^+$ = 504

Beispiel 12

4'-[[2-n-Propyl-4-methyl-6-(2,2-dicyano-1-dimethylamino-ethenylenamino)-1H-benzimidazol-1-yl]-methyl]-biphenyl-2-carbonsäure

a) 4'-[[2-n-Propyl-4-methyl-6-(2,2-dicyano-1-dimethylamino-ethenylenamino)-1H-benzimidazol-1-yl]-methyl]-biphenyl-2-carbonsäure-tert.butylester

Hergestellt analog Beispiel 10d aus 4'-[[2-n-Propyl-4-methyl-6-(2,2-dicyano-1-methylmercapto-ethenylenamino)-1H-benzimidazol-1-yl]-methyl]-biphenyl-2-carbonsäure-tert.butylester und Dimethylamin.
Ausbeute: 38 % der Theorie,
Schmelzpunkt: sintert ab 138 ° C

b) 4'-[[2-n-Propyl-4-methyl-6-(2,2-dicyano-1-dimethylamino-ethenylenamino)-1H-benzimidazol-1-yl]-methyl]-biphenyl-2-carbonsäure

Hergestellt analog Beispiel 10e aus 4'-[[2-n-Propyl-4-methyl-6-(2,2-dicyano-1-dimethylamino-ethenylenamino)-1H-benzimidazol-1-yl]-methyl]-biphenyl-2-carbonsäure-tert.butylester und Trifluoressigsäure.
Ausbeute: 49 % der Theorie,
Schmelzpunkt: 178 ° C
$C_{31}H_{30}N_6O_2$ (518,62)
Massenspektrum: $M^+$ = 519

Beispiel 13

4'-[[2-n-Propyl-4-methyl-6-(2,2-dicyano-1-dimethylamino-ethenylenamino)-1H-benzimidazol-1-yl]-methyl]-2-(1H-tetrazol-5-yl)-biphenyl

a) 4'-[[2-n-Propyl-4-methyl-6-(2,2-dicyano-1-methylmercapto-ethenylenamino)-1H-benzimidazol-1-yl]-methyl]-2-(1H-tetrazol-5-yl)-biphenyl

Hergestellt analog Beispiel 10c aus 4'-[[2-n-Propyl-4-methyl-6-amino-1H-benzimidazol-1-yl)-methyl]-2-(1H-tetrazol-5-yl)-biphenyl und 2,2-Dicyano-1,1-dimethylmercapto-ethen.
Ausbeute: 79 % der Theorie,

Schmelzpunkt: 184-185 °C

b) 4'-[[2-n-Propyl-4-methyl-6-(2,2-dicyano-1-dimethylamino-ethenylenamino)-1H-benzimidazol-1-yl]-methyl]-2-(1H-tetrazol-5-yl)-biphenyl

Hergestellt analog Beispiel 10d aus 4'-[[2-n-Propyl-4-methyl-6-(2,2-dicyano-1-methylmercapto-ethenylenamino)-1H-benzimidazol-1-yl]-methyl]-2-(1H-tetrazol-5-yl)-biphenyl und Dimethylamin.
Ausbeute: 29 % der Theorie,
Schmelzpunkt: sintert ab 208 °C
$C_{31}H_{30}N_{10}$ (542,65)
Massenspektrum: $(M+H)^+ = 543$

Beispiel 14

4'-[[2-n-Propyl-4-methyl-6-(1-dimethylamino-2-nitro-ethenylenamino)-1H-benzimidazol-1-yl]-methyl]-biphenyl-2-carbonsäure

a)4'-[[2-n-Propyl-4-methyl-6-(1-methylmercapto-2-nitroethenylenamino)-1H-benzimidazol-1-yl]-methyl]-biphenyl-2-carbonsäure-methylester

826 mg (2,0 mMol) 4'-[(2-n-Propyl-4-methyl-6-amino-1H-benzimidazol-1-yl)-methyl]-biphenyl-2-carbonsäure-methylester werden in 75 ml Isopropanol gelöst, mit 0,97 g (6,0 mMol) 1,1-Bis-(methylmercapto)-2-nitro-ethen versetzt und 6 Stunden unter Rückfluß erhitzt. Danach wird das Solvens abgedampft, der Rückstand wird aus Isopropanol/Petrolether (3:1) unter Zusatz von Aktivkohle umkristallisiert.
Ausbeute: 745 mg (70 % der Theorie),
Schmelzpunkt: 173-174 °C

b) 4'-[[2-n-Propyl-4-methyl-6-(1-dimethylamino-2-nitroethenylenamino)-1H-benzimidazol-1-yl]-methyl]-biphenyl-2-carbonsäure-methylester

250 mg (0,47 mMol) 4'-[(2-n-Propyl-4-methyl-6-(1-methylmercapto-2-nitro-ethenylenamino)-1H-benzimidazol-1-yl]-methyl]-biphenyl-2-carbonsäure-methylester werden in 50 ml Isopropanol gelöst, mit 1,0 ml Dimethylamin versetzt und in in einem Druckgefäß 3 Stunden auf 60 °C erhitzt. Nach Abkühlung auf Raumtemperatur wird gebildeter Niederschlag abfiltriert, mit Wasser und Ether gewaschen und getrocknet.
Ausbeute: 195 mg (79 % der Theorie),
Schmelzpunkt: 127-128 °C

c) 4'-[[2-n-Propyl-4-methyl-6-(1-dimethylamino-2-nitroethenylenamino)-1H-benzimidazol-1-yl]-methyl]-biphenyl-2-carbonsäure

Hergestellt analog Beispiel 1d aus 4'-[[2-n-Propyl-4-methyl-6-(1-dimethylamino-2-nitro-ethenylenamino)-1H-benzimid azol-1-yl]-methyl]-biphenyl-2-carbonsäure-methylester und 2N Natronlauge in Ethanol.
Ausbeute: 34 % der Theorie,
Schmelzpunkt: sintert ab 200 °C
$C_{29}H_{31}N_5O_4$ (513,60)
Massenspektrum: $M^+ = 513$

Beispiel 15

4'-[[2-n-Propyl-4-methyl-6-(1-methylamino-2-nitro-ethenylenamino)-1H-benzimidazol-1-yl]-methyl]-biphenyl-2-carbonsäure

a) 4'-[[2-n-Propyl-4-methyl-6-(1-methylamino-2-nitro-ethenylenamino)-1H-benzimidazol-1-yl]-methyl]-biphenyl-2-carbonsäure-methylester

Hergestellt analog Beispiel 14b aus 4'-[[2-n-Propyl-4-methyl-6-(1-methylmercapto-2-nitro-ethenylenamino)-1H-benzimidazol-1-yl]-methyl]-biphenyl-2-carbonsäure-methylester und Methylamin.
Ausbeute: 68 % der Theorie,

$R_f$-Wert: 0,15 (Kieselgel; Methylenchlorid/Ethanol = 9:1)

b) 4'-[[2-n-Propyl-4-methyl-6-(1-methylamino-2-nitro-ethenylenamino)-1H-benzimidazol-1-yl]-methyl]-biphenyl-2-carbonsäure

Hergestellt analog Beispiel 1d aus 4'-[[2-n-Propyl-4-methyl-6-(1-methylamino-2-nitro-ethenylenamino)-1H-benzimidazol-1-yl]-methyl]-biphenyl-2-carbonsäure-methylester und 2N Natronlauge in Ethanol.
Ausbeute: 30 % der Theorie,
Schmelzpunkt: sintert ab 188 °C
$C_{28}H_{29}N_5O_4$ (499,57)
Massenspektrum: $M^+$ = 499

Beispiel 16

4'-[[2-n-Propyl-4-methyl-6-(3-methyl-2-amidosulfonyl-guanidino)-1H-benzimidazol-1-yl]-methyl]-biphenyl-2-carbonsäure

a) 4'-[[2-n-Propyl-4-methyl-6-(3-methyl-2-amidosulfonyl-guanidino)-1H-benzimidazol-1-yl]-methyl]-biphenyl-2-carbonsäure-methylester

413 mg (1,0 mMol) 4'-[(2-n-Propyl-4-methyl-6-amino-1H-benzimidazol-1-yl)-methyl]-biphenyl-2-carbonsäure-methylester werden in 50 ml Isopropanol gelöst, mit 1,17 g (4,0 mMol) Amidosulfonylcarbamidsäurediphenylester versetzt und 2 Tage bei Raumtemperatur gerührt. Nach Zusatz von 1,5 ml Methylamin wird das Reaktionsgemisch in einem Druckgefäß 3 Stunden auf 60 °C erhitzt. Danach wird das Solvens abgedampft, der Rückstand wird an Kieselgel (Korngröße: 0,032-0,063 mm) chromatographiert, wobei als Elutionsmittel anfangs Methylenchlorid, später Methylenchlorid/Ethanol (50:1, 25:1 und 19:1) verwendet wird. Die einheitlichen Fraktionen werden vereinigt und eingedampft, der Rückstand wird aus Ether/Petrolether (1:1) verrieben und abgesaugt.
Ausbeute: 235 mg (43 % der Theorie),
Schmelzpunkt: 115-117 °C

b) 4'-[[2-n-Propyl-4-methyl-6-(3-methyl-2-amidosulfonyl-guanidino)-1H-benzimidazol-1-yl]-methyl]-biphenyl-2-carbonsäure

Hergestellt analog Beispiel 1d aus 4'-[[2-n-Propyl-4-methyl-6-(3-methyl-2-amidosulfonyl-guanidino)-1H-benzimidazol-1-yl]-methyl]-biphenyl-2-carbonsäure-methylester und 2N Natronlauge in Ethanol.
Ausbeute: 31 % der Theorie,
Schmelzpunkt: 208 °C
$C_{27}H_{30}N_6O_4S$ (534,64)
Massenspektrum: $(M+H)^+$ = 535

Beispiel 17

4'-[[2-n-Propyl-4-methyl-6-(3,3-dimethyl-2-amidosulfonylguanidino)-1H-benzimidazol-1-yl]-methyl]-biphenyl-2-carbonsäure

a) 4'-[[2-n-Propyl-4-methyl-6-(3,3-dimethyl-2-amidosulfonylguanidino)-1H-benzimidazol-1-yl]-methyl]-biphenyl-2-carbonsäure-methylester

Hergestellt analog Beispiel 16a aus 4'-[(2-n-Propyl-4-methyl-6-amino-1H-benzimidazol-1-yl)-methyl]-biphenyl-2-carbonsäure-methylester, Amidosulfonylcarbamidsäure-diphenylester und Dimethylamin.
Ausbeute: 56 % der Theorie,
Schmelzpunkt: 188-189 °C

b) 4'-[[2-n-Propyl-4-methyl-6-(3,3-dimethyl-2-amidosulfonylguanidino)-1H-benzimidazol-1-yl]-methyl]-biphenyl-2-carbonsäure

Hergestellt analog Beispiel 1d aus 4'-[[2-n-Propyl-4-methyl-6-(3,3-dimethyl-2-amidosulfonyl-guanidino)-1H-benzimidazol-1-yl]-methyl]-biphenyl-2-carbonsäure-methylester und 2N Natronlauge in Ethanol.
Ausbeute: 26 % der Theorie,
Schmelzpunkt: sintert ab 165 °C
$C_{28}H_{32}N_6O_4S$ (548,67)
Massenspektrum: $(M+H)^+ = 549$

Beispiel 18

4'-[[2-n-Propyl-4-methyl-6-[2-cyano-3-(2-dimethylamino-ethyl)-3-methyl-guanidino]-1H-benzimidazol-1-yl]-methyl]-2-(1H-tetrazol-5-yl)-biphenyl

Hergestellt analog Beispiel 6d aus 4'-[[2-n-Propyl-4-methyl-6-(2-phenoxy-3-cyano-isoureido)-1H-benzimidazol-1-yl]-methyl]-2-(1H-tetrazol-5-yl)-biphenyl und N-(2-Dimethylamino-ethyl)-methylamin in Isopropanol.
Ausbeute: 40 % der Theorie,
Schmelzpunkt: 220 °C (sintert ab 115 °C)
$C_{32}H_{37}N_{11}$ (575,74)
Massenspektrum: $(M+H)^+ = 576$

Beispiel 19

4'-[[2-n-Propyl-4-methyl-6-[2-cyano-3-(2-hydroxy-ethyl)-3-methyl-guanidino]-1H-benzimidazol-1-yl]-methyl]-2-(1H-tetrazol-5-yl)-biphenyl

Hergestellt analog Beispiel 6d aus 4'-[[2-n-Propyl-4-methyl-6-(2-phenoxy-3-cyano-isoureido)-1H-benzimidazol-1-yl)-methyl]-2-(1H-tetrazol-5-yl)-biphenyl und N-(2-Hydroxy-ethyl)-methylamin in Isopropanol.
Ausbeute: 47,5 % der Theorie,
Schmelzpunkt: 230 °C (sintert ab 108 °C)
$C_{30}H_{32}N_{10}O$ (548,67)
Massenspektrum: $(M+H)^+ = 549$

Beispiel 20

4'-[[2-n-Propyl-4-methyl-6-[2-cyano-3-(bis-2-methoxy-ethyl)-guanidino]-1H-benzimidazol-1-yl]-methyl]-2-(1H-tetrazol-5-yl)-biphenyl

Hergestellt analog Beispiel 6d aus 4'-[[2-n-Propyl-4-methyl-6-(2-phenoxy-3-cyano-isoureido)-1H-benzimidazol-1-yl]-methyl]-2-(1H-tetrazol-5-yl)-biphenyl und Bis-(2-methoxy)-ethylamin in Isopropanol.
Ausbeute: 27,5 % der Theorie,
Schmelzpunkt: 104-106 °C (sintert ab 70 °C),
$C_{33}H_{38}N_{10}O_2$ (606,74)
Massenspektrum: $M^+ = 606$

Beispiel 21

4'-[[2-n-Propyl-4-methyl-6-(N-(2-methyl-1-cyanimino-propyl)-amino)-1H-benzimidazol-1-yl]-methyl]-2-(1H-tetrazol-5-yl)-biphenyl

a) (1-Methyl-ethyl)-(methylmercapto)-methyleniminonitril

Eine aus 28,0 g (0,23 Mol) Isopropylbromid und 5,5 g (0,23 Mol) Magnesium in 10 ml Diethylether hergestellte Grignardlösung wird innerhalb von 30 Minuten unter Eiskühlung zu einer Lösung von 22,0 g (0,15 Mol) Cyanimidodithiokohlensäuredimethylester in 150 ml Tetrahydrofuran getropft. Die Lösung wird 24 Stunden bei Raumtemperatur gerührt und anschließend mit 100 ml 4N Salzsäure versetzt. Nach der

Trennung der Phasen wird die wässrige Phase 3 x mit je 50 ml Essigester extrahiert. Die vereinigten organischen Phasen werden mit Kochsalzlösung gewaschen und mit Natriumsulfat getrocknet. Das Rohprodukt wird an Kieselgel (Korngröße: 0,032-0,063 mm) chromatographiert, wobei anfangs Petrolether, später Gemische aus Petrolether/Essigester (19:1, 9:1 und 4:1) verwendet werden. Die einheitlichen Fraktionen werden vereinigt und eingedampft.
Ausbeute: 4,65 g (22 % der Theorie),
$R_f$: 0,40 (Kieselgel; Petrolether/Essigester = 9:1)

b) 4'-[[2-n-Propyl-4-methyl-6-(N-(2-methyl-1-cyanimino-propyl)-amino)-1H-benzimidazol-1-yl]-methyl]-2-(1H-tetrazol-5-yl)-biphenyl

Hergestellt analog Beispiel 1c aus 4'-[(2-n-Propyl-4-methyl-6-amino-1H-benzimidazol-1-yl]-methyl]-2-(1H-tetrazol-5-yl)-biphenyl und (1-Methyl-ethyl)-(methylmercapto)-methylen-iminonitril in Isopropanol.
Ausbeute: 20,3 % der Theorie,
Schmelzpunkt: 208-210 °C (sintert ab 162 °C),
$C_{30}H_{31}N_9$ (517,65)
Massenspektrum: $M^+$ = 517

Beispiel 22

4'-[[2-n-propyl-4-methyl-6-[2-cyano-3-(2-hydroxy-ethyl)-3-methyl-guanidino]-1H-benzimidazol-1-yl]-methyl]-biphenyl-2-carbonsäure

Hergestellt analog Beispiel 1d aus 4'-[[2-n-Propyl-4-methyl-6-[2-cyano-3-(2-hydroxy-ethyl)-3-methyl-guanidino]-1H-benzimidazol-1-yl]-methyl]-biphenyl-2-carbonsäure-methylester und 2N Natronlauge in Ethanol.
Ausbeute: 9,5 % der Theorie,
Schmelzpunkt: 212 °C (sintert ab 190 °C)
$C_{30}H_{32}N_6O_3$ (524,64)
Massenspektrum: $(M-H)^-$ = 523

Beispiel 23

4'-[[2-n-Propyl-4-methyl-6-[2-cyano-3,3-[bis(2-diethylaminoethyl)]-guanidino]-1H-benzimidazol-1-yl]-methyl]-biphenyl-2-carbonsäure

Hergestellt analog Beispiel 1d aus 4'-[[2-n-Propyl-4-methyl-6-[2-cyano-3,3-[bis(2-diethylamino-ethyl)]-guanidino]-1H-benzimidazol-1-yl]-methyl]-biphenyl-2-carbonsäure-methylester und 2N Natronlauge in Ethanol.
Ausbeute: 28,8 % der Theorie,
Schmelzpunkt: 168 °C (sintert ab 112 °C)
$C_{39}H_{51}N_8O_2$ (663,89)
Massenspektrum: $(M-H)^-$ = 663

Beispiel 24

4'-[[2-Ethyl-4-methyl-6-[2-cyano-3-(4-methyl-piperazino)-amidino]-1H-benzimidazol-1-yl]-methyl]-biphenyl-2-carbonsäure

Hergestellt analog Beispiel 1d aus 4'-[[2-Ethyl-4-methyl-6-[2-Cyano-3-(4-methyl-piperazino)-amidino]-1H-benzimidazol-1-yl]-methyl]-biphenyl-2-carbonsäure-methylester und 2N Natronlauge in Ethanol.
Ausbeute: 3,9 % der Theorie,
Schmelzpunkt: 218 °C (sintert ab 173 °C)
$C_{31}H_{33}N_7O_2$ (535,66)
Massenspektrum: $(M+H)^+$ = 536

Beispiel 25

4'-[[2-Ethyl-4-methyl-6-(2-cyano-3,3-dimethyl-guanidino)-1H-benzimidazol-1-yl]-methyl]-2-(1H-tetrazol-5-yl)-biphenyl

Hergestellt analog Beispiel 6d aus 4'-[[2-Ethyl-4-methyl-6-(2-phenoxy-3-cyano-isoureido)-1H-benzimidasol-1-yl]-methyl]-2-(1H-tetrazol-5-yl)-biphenyl und Dimethylamin in Isopropanol.
Ausbeute: 17,8 % der Theorie,
Schmelzpunkt: sintert ab 202 °C
$C_{28}H_{28}N_{10}$ (504,61)
Massenspektrum: $M^{+}$ = 504

Beispiel 26

4'-[[2-Ethyl-4-methyl-6-(2-cyano-3,3-dimethyl-guanidino)-1H-benzimidazol-1-yl]-methyl]-biphenyl-2-carbonsäure

Hergestellt analog Beispiel 1d aus 4'-[[2-Ethyl-4-methyl-6-(2-cyano-3,3-dimethyl-guanidino)-1H-benzimidazol-1-yl]-methyl]-biphenyl-2-carbonsäure-methylester und 2N Natronlauge in Ethanol.
Ausbeute: 25,0 % der Theorie,
Schmelzpunkt: 208-210 °C (sintert ab 176 °C)
$C_{28}H_{28}N_{6}O_{2}$ (480,58)
Massenspektrum: $M^{+}$ = 480

Beispiel 27

4'-[[2-n-Propyl-4-methyl-6-[1-(N-(2-dimethylamino-ethyl)-methylamino)-2-nitro-ethylenamino]-1H-benzimidazol-1-yl]-methyl]-biphenyl-2-carbonsäure

Hergestellt analog Beispiel 1d aus 4'-[[2-n-Propyl-4-methyl-6-[1-(N-(2-dimethylamino-ethyl)-methylamino)-2-nitro-ethylenamino]-1H-benzimidazol-1-yl]-methyl]-biphenyl-2-carbonsäure-methylester und 2N Natronlauge in Ethanol.
Ausbeute: 25,0 % der Theorie,
Schmelzpunkt: 218 °C (sintert ab 202 °C)
$C_{32}H_{38}N_{6}O_{4}$ (570,70)
Massenspektrum: $(M-H)^{-}$ = 569

Beispiel 28

4'-[[2-n-Propyl-4-methyl-6-(1,1-dimethylamino-2-nitro-ethylenamino)-1H-benzimidazol-1-yl]-methyl]-2-(1H-tetrazol-5-yl)-biphenyl

Hergestellt analog Beispiel 14b aus 4'-[(2-n-Propyl-4-methyl-6-(1-methylmercapto-2-nitro-ethylenamino-1H-benzimidazol-1-yl)-methyl]-2-(1H-tetrazol-5-yl)-biphenyl und Dimethylamin.
Ausbeute: 9,5 % der Theorie,
Schmelzpunkt: sintert ab 172 °C
$C_{29}H_{31}N_{9}O_{2}$ (537,64)
Massenspektrum: $(M+H)^{+}$ = 538

Beispiel 29

4'-[[2-Ethyl-4-methyl-6-(1,1-dimethylamino-2-nitro-ethylenamino)-1H-benzimidazol-1-yl]-methyl]-2-(1H-tetrazol-5-yl)-biphenyl

Hergestellt analog Beispiel 14b aus 4'-[(2-Ethyl-4-methyl-6-(1-methylmercapto-2-nitro-ethylenamino-1H-benzimidazol-1-yl)-methyl]-2-(1H-tetrazol-5-yl)-biphenyl und Dimethylamin.
Ausbeute: 23,1 % der Theorie,
Schmelzpunkt: sintert ab 196 °C

$C_{28}H_{29}N_9O_2$ (523,61)
Massenspektrum: $(M+H)^+$ = 524

Beispiel 30

4'-[[2-n-Propyl-4-methyl-6-(1-morpholino-2-nitro-ethylenamino)-1H-benzimidazol-1-yl]-methyl]-2-(1H-tetrasol-5-yl)-biphenyl

Hergestellt analog Beispiel 14b aus 4'-[[2-n-Propyl-4-methyl-6-(1-methylmercapto-2-nitro-ethylenamino)-1H-benzimidazol-1-yl]-2-(1H-tetrazol-5-yl)-biphenyl und Morpholin.
Ausbeute: 9,2 % der Theorie,
Schmelzpunkt: 178 °C (sintert ab 118 °C)
$C_{31}H_{33}N_9O_3$ (579,67)
Massenspektrum: $(M+H)^+$ = 580

Beispiel 31

4'-[[2-n-Propyl-4-methyl-6-[1-(N-(2-dimethylamino-ethyl)-methylamino)-2-nitro-ethylenamino]-1H-benzimidazol-1-yl]-methyl]-2-(1H-tetrazol-5-yl)-biphenyl

Hergestellt analog Beispiel 14b aus 4'-[[2-n-Propyl-4-methyl-6-(1-methylmercapto-2-nitro-ethylenamino]-1H-benzimidazol-1-yl]-methyl]-2-(1H-tetrazol-5-yl)-biphenyl und 2-Dimethylamino-methylamin.
Ausbeute: 9,2 % der Theorie,
Schmelzpunkt: 182 °C (sintert ab 145 °C)
$C_{32}H_{38}N_{10}O_2$ (594,73)
Massenspektrum: $(M+H)^+$ = 595

Beispiel 32

4'-[[2-Ethyl-4-methyl-6-[(2-dimethylamino-3,4-dioxo-1-cyclobuten-1-yl)-amino]-1H-benzimidazol-1-yl]-methyl]-biphenyl-2-carbonsäure

a) 4'-[[2-Ethyl-4-methyl-6-[(2-dimethylamino-3,4-dioxo-1-cyclobuten-1-yl)-amino]-1H-benzimidazol-1-yl]-methyl]-biphenyl-2-carbonsäure-methylester

Hergestellt analog Beispiel 1c aus 4'-[[2-Ethyl-4-methyl-6-amino-1H-benzimidazol-1-yl]-methyl]-biphenyl-2-carbonsäure-methylester, 3,4-Diethoxy-3-cyclobuten-1,2-dion und Dimethylamin in Isopropanol.
Ausbeute: 74 % der Theorie,
Harz, $R_f$-Wert 0,40 (Kieselgel, Methylenchlorid/Methanol = 9:1)

b) 4'-[[2-Ethyl-4-methyl-6-[(2-dimethylamino-3,4-dioxo-1-cyclobuten-1-yl)-amino]-1H-benzimidazol-1-yl]-methyl]-biphenyl-2-carbonsäure

Hergestellt analog Beispiel 1d aus 4'-[[2-Ethyl-4-methyl-6-[(2-dimethylamino-3,4-dioxo-1-cyclobuten-1-yl)-amino]-1H-benzimidazol-1-yl]-methyl]-biphenyl-2-carbonsäure-methylester und 1N Natronlauge in Ethanol.
Ausbeute: 26,5 % der Theorie,
Schmelzpunkt: 312-315 °C
$C_{30}H_{28}N_4O_4$ (508,60)
Massenspektrum: $M^+$ = 508

Beispiel 33

4'-[[2-Ethyl-4-methyl-6-[(2-dimethylamino-3,4-dioxo-1-cyclobuten-1-yl)-amino]-1H-benzimidazol-1-yl]-methyl]-2-(1H-tetrazol-5-yl)-biphenyl

Hergestellt analog Beispiel 14b aus 4'-[[2-Ethyl-4-methyl-6-[(2-ethoxy-3,4-dioxo-1-cyclobuten-1-yl)-amino]-1H-benzimid azol-1-yl]-methyl]-2-(1H-tetrazol-5-yl)-biphenyl und Dimethylamin.

Ausbeute: 38,8 % der Theorie,
Schmelzpunkt: 286-290 °C
$C_{30}H_{28}N_8O_2$ (532,61)
Massenspektrum: $(M+H)^+ = 533$

Beispiel 34

4'-[[2-Ethyl-4-methyl-6-(3,3-dimethyl-2-methylsulfonyl-guanidino)-1H-benzimidazol-1-yl]-methyl]-biphenyl-2-carbonsäure

Hergestellt analog Beispiel 1d aus 4'-[[2-Ethyl-4-methyl-6-(3,3-dimethyl-2-methylsulfonyl-guanidino)-1H-benzimidazol-1-yl]-methyl]-biphenyl-2-carbonsäure-methylester und 2N Natronlauge in Ethanol.
Ausbeute: 47,9 % der Theorie,
Schmelzpunkt: 204-206 °C
$C_{28}H_{31}N_5O_4S$ (533,60)
Massenspektrum: $(M-H)^- = 532$

Beispiel 35

4'-[[2-Ethyl-4-methyl-6-(3,3-dimethyl-2-methylsulfonyl-guanidino)-1H-benzimidazol-1-yl]-methyl]-2-(1H-tetrazol-5-yl)-biphenyl

Hergestellt analog Beispiel 14b aus 4'-[[2-Ethyl-4-methyl-6-(2-phenoxy-3-methylsulfonyl-isoureido)-1H-benzimidazol-1-yl]-methyl]-2-(1H-tetrazol-5-yl)-biphenyl und Dimethylamin.
Ausbeute: 58,0 % der Theorie,
Schmelzpunkt: 197-199 °C (sintert ab 172 °C)
$C_{28}H_{31}N_9O_2S$ (557,69)
Massenspektrum: $(M+H)^+ = 558$

Beispiel 36

4'-[[2-Ethyl-4-methyl-6-(3,3-dimethyl-2-phenylsulfonyl-guanidino)-1H-benzimidazol-1-yl]-methyl]-biphenyl-2-carbonsäure

Hergestellt analog Beispiel 1d aus 4'-[[2-Ethyl-4-methyl-6-(3,3-dimethyl-2-phenylsulfonyl-guanidino)-1H-benzimidazol-1-yl]-methyl]-biphenyl-2-carbonsäure-methylester und 2N Natronlauge in Ethanol.
Ausbeute: 30,8 % der Theorie,
Schmelzpunkt: 218-220 °C
$C_{33}H_{33}N_5O_4S$ (595,70)
Massenspektrum: $(M+H)^+ = 596$

Beispiel 37

4'-[[2-n-Propyl-4-methyl-6-[N-(3-nitro-pyrrol-2-yl)-amino]-1H-benzimidazol-1-yl]-methyl]-biphenyl-2-carbonsäure

a) 4'-[[2-n-Propyl-4-methyl-6-[1-(2-diethoxy-ethylamino)-2-nitro-ethylenamino)-1H-benzimidazol-1-yl]-methyl]-biphenyl-2-carbonsäure-methylester

Hergestellt analog Beispiel 14b aus 4'-[[2-n-Propyl-4-methyl-6-(1-methylmercapto-2-nitro-ethylenamino)-1H-benzimidazol-1-yl]-methyl]-biphenyl-2-carbonsäure-methylester und Aminoacetaldehyd-diethylacetal.
Ausbeute: 32 % der Theorie,
Öl, $R_f$-Wert: 0,50 (Kieselgel; Methylenchlorid/Ethanol = 19:1)

b) 4'-[[2-n-Propyl-4-methyl-6-[N-(3-nitro-pyrrol-2-yl)-amino]-1H-benzimidazol-1-yl]-methyl]-biphenyl-2-carbonsäure-methylester

190 mg (0,3 mMol) 4'-[[2-n-Propyl-4-methyl-6-[1-(2-diethoxy)-ethylamino-2-nitro-ethylenamino)-1H-benzimidazol-1-yl]-methyl]-biphenyl-2-carbonsäure-methylester werden in 50 ml 0,05N Salzsäure 30 Minuten auf dem Dampfbad erhitzt. Nach dem Abkühlen wird der pH-Wert mit Natriumcarbonatlösung auf pH 8 eingestellt und 3 mal mit Essigester extrahiert. Die vereinigten organischen Phasen werden mit Kochsalzlösung gewaschen und über Natriumsulfat getrocknet. Nach Eindampfen wird der erhaltene Rückstand an Kieselgel chromatographiert, wobei anfangs Methylenchlorid, später Methylenchlorid/Ethanol 25:1 und 19:1 verwendet wird. Die einheitlichen Fraktionen werden vereinigt und eingedampft.
Ausbeute: 105 mg (65 % der Theorie),
Öl, $R_f$-Wert: 0,50 (Kieselgel; Methylenchlorid/Ethanol = 19:1)

c) 4'-[[2-n-Propyl-4-methyl-6-[N-(3-nitro-pyrrol-2-yl)-aminol-1H-benzimidazol-1-yl]-methyl]-biphenyl-2-carbonsäure

Hergestellt analog Beispiel 1d aus 4'-[[2-n-Propyl-4-methyl-6-[N-(3-nitro-pyrrol-2-yl)-amino]-1H-benzimidazol-1-yl]-methyl]-biphenyl-2-carbonsäure-methylester und 2N Natronlauge in Ethanol.
Ausbeute: 16,9 % der Theorie,
Schmelzpunkt: sintert ab 165 ° C
$C_{29}H_{27}N_5O_4$ (509,60)
Massenspektrum: $(M-H)^-$ = 508

Beispiel 38

4'-[[2-Ethyl-4-methyl-6-(3,3-dimethyl-2-cyano-1-methyl-guanidino)-1H-benzimidazol-1-yl]-methyl]-biphenyl-2-carbonsäure

Hergestellt analog Beispiel 1d aus 4'-[[2-Ethyl-4-methyl-6-(3,3-dimethyl-2-cyano-1-methyl-guanidino)-1H-benzimidazol-1-yl]-methyl]-biphenyl-2-carbonsäure-methylester und 2N Natronlauge in Ethanol.
Ausbeute: 12,9 % der Theorie,
Schmelzpunkt: 182-184 ° C (sintert ab 135 ° C)
$C_{29}H_{30}N_6O_2$ (494,61)
Massenspektrum: $M^+$ = 494

Beispiel 39

4'-[[2-n-Propyl-4-chlor-6-(1-dimethylamino-2-nitro-ethylenamino)-1H-benzimidazol-1-yl]-methyl]-2-(1H-tetrazol-5-yl)biphenyl-Kaliumsalz

Hergestellt analog Beispiel 14b aus 4'-[[2-n-Propyl-4-chlor-6-(1-methylmercapto-2-nitro-ethylenamino)-1H-benzimidazol-1-yl]-methyl]-2-(1H-tetrazol-5-yl)biphenyl und Dimethylamin. Die so erhaltene Verbindung wird anschließend in alkoholischer Lösung mit Kalilauge in das entsprechende Kaliumsalz übergeführt.
Ausbeute: 47,2 % der Theorie,
Schmelzpunkt: sintern ab 118 ° C
$C_{28}H_{27}ClKN_9O_2$ (596,16)
Massenspektrum: $(M+H)^+$ = 596/598 (Cl)

Beispiel 40

4'-[[2-n-Propyl-4-chlor-6-(2,2-dicyano-1-dimethylamino-ethylenamino)-1H-benzimidazol-1-yl]-methyl]-2-(1H-tetrazol-5-yl)-biphenyl-Kaliumsalz

Hergestellt analog Beispiel 10d 4'-[[2-n-Propyl-4-chlor-6-(2,2-dicyano-1-methylmercapto-ethylenamino)-1H-benzimidazol-1-yl]-methyl]-2-(1H-tetrazol-5-yl)biphenyl und Dimethylamin in Gegenwart von 3-Chlorperoxybenzoesäure. Die so erhaltene Verbindung wird anschließend in alkoholischer Lösung mit Kalilauge in das entsprechende Kaliumsalz übergeführt.
Ausbeute: 52,5 % der Theorie,

Schmelzpunkt: 210-212 °C (sintert ab 177 °C)
$C_{30}H_{27}ClKN_{10}$ (563,09)
Massenspektrum: $(M+H)^+$ = 563/565 (Cl)
Bei den nachfolgenden pharmazeutischen Anwendungsbeispielen kann als Wirksubstanz jede geeignete Verbindung der Formel I, insbesondere diejenigen, in denen $R_d$ eine Carboxy- oder 1H-Tetrazolylgruppe darstellt, eingesetzt werden:

Beispiel I

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel II

| Dragees, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1.0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel III

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45 °C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel IV

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel V

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 °C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VI

| Suppositorien, enthaltend 100 mg Wirkstoff | |
|---|---|
| Wirkstoff | 100,0 mg |
| Adeps solidus | 1600.0 mg |
| | 1700,0 mg |

Herstellung:

Das Hartfett wird geschmolzen. Bei 40 °C wird die gemahlene Wirksubstanz in der Schmelze homogen dispergiert. Es wird auf 38 °C abgekühlt und in schwach vorgekühlte Suppositorienformen ausgegossen.

**Patentansprüche**

**1.** Benzimidazole der allgemeinen Formel

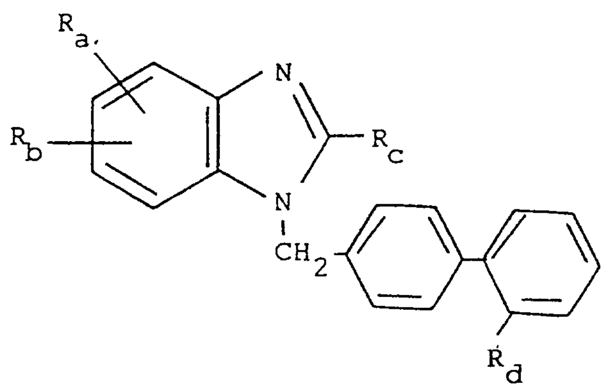

,(I)

in der

$R_a$ eine Alkylgruppe mit 1 bis 3 Kohlenstoffatomen, eine Trifluormethylgruppe, ein Wasserstoff-, Fluor-, Chlor- oder Bromatom,

$R_b$ eine $R_1NH$-Gruppe, in der das Wasserstoffatom durch eine R-CX-Gruppe, durch eine in 2-Stellung durch eine Alkoxygruppe mit 1 bis 3 Kohlenstoffatomen oder durch eine $(R_1NR_3)$-Gruppe substituierte 3,4-Dioxo-1-cyclobuten-1-yl-Gruppe oder durch eine 3-Nitro-pyrrol-2-yl-Gruppe ersetzt ist, wobei die Pyrrolgruppe in 1-Stellung zusätzlich durch eine Alkylgruppe mit 1 bis 3 Kohlenstoffatomen substituiert sein kann, in denen

R eine Alkylgruppe mit 1 bis 5 Kohlenstoffatomen, eine Alkoxy- oder Alkylthiogruppe mit jeweils 1 bis 3 Kohlenstoffatomen, eine Phenoxy- oder Phenylthiogruppe oder eine $(R_2NR_3)$-Gruppe und

X eine $NO_2$-CH=, $(R_4R_5)C$= oder $R_6$-N= Gruppe, in denen

$R_1$ ein Wasserstoffatom oder eine Alkylgruppe mit 1 bis 3 Kohlenstoffatomen,

$R_2$ ein Wasserstoffatom, eine verzweigte oder unverzweigte Alkylgruppe mit 1 bis 10 Kohlenstoffatomen, die in 2-, 3- oder 4-Stellung durch eine Hydroxy-, Alkoxy-, Amino-, Alkylamino- oder Dialkylaminogruppe substituiert sein kann, in denen der Alkylteil jeweils 1 bis 3 Kohlenstoffatome enthalten kann, eine Alkylgruppe mit 1 bis 4 Kohlenstoffatomen, die durch eine Phenyl- oder Pyridylgruppe substituiert ist und zusätzlich in 2-, 3- oder 4-Stellung durch eine Hydroxygruppe substituiert sein kann, eine Cycloalkylgruppe mit 3 oder 4 Kohlenstoffatomen, eine Cycloalkylgruppe mit 5 bis 8

Kohlenstoffatomen, in der eine Ethylenbrücke durch eine o-Phenylengruppe ersetzt sein kann, eine gegebenenfalls durch 1, 2 oder 3 Alkylgruppen substituierte Bicycloalkylgruppe mit 6 bis 8 Kohlenstoffatomen,

$R_3$ ein Wasserstoffatom oder eine Alkylgruppe, die in 2- oder 3-Stellung durch eine Alkoxy-, Amino-, Alkylamino- oder Dialkylaminogruppe substituiert sein kann, wobei die vorstehend erwähnten Alkyl- und Alkoxyteile jeweils 1 bis 3 Kohlenstoffatome enthalten können, oder

$R_2$ und $R_3$ zusammen mit dem dazwischenliegenden Stickstoffatom eine cyclische Alkyleniminogruppe mit 4 bis 6 Kohlenstoffatomen, die durch ein oder zwei Alkylgruppen oder durch eine Phenylgruppe substituiert sein kann und in der zusätzlich eine Ethylenbrücke in 3,4-Stellung durch eine o-Phenylengruppe ersetzt sein kann, eine Morpholinogruppe oder eine gegebenenfalls in 4-Stellung durch eine Alkylgruppe mit 1 bis 3 Kohlenstoffatomen oder durch eine Phenylgruppe substituierte Piperazinogruppe,

$R_4$ und $R_5$, die gleich oder verschieden sein können, jeweils eine Cyano-, $R_2NR_3$-CO- oder Alkoxycarbonylgruppe mit insgesamt 2 bis 5 Kohlenstoffatomen, wobei $R_2$ und $R_3$ wie vorstehend erwähnt definiert sind, und

$R_6$ eine Cyano-, Tetrazol-5-yl-, Alkansulfonyl-, Phenylsulfonyl-, Phenylalkansulfonyl-, Aminosulfonyl-, Alkylaminosulfonyl-, Dialkylaminosulfonyl-, Alkylcarbonyl-, Phenylcarbonyl-, Aminocarbonyl-, Alkylaminocarbonyl- oder Dialkylaminocarbonylgruppe, in denen der Alkylteil jeweils 1 bis 3 Kohlenstoffatome enthalten kann, darstellen,

$R_c$ eine Alkylgruppe mit 2 bis 4 Kohlenstoffatomen, eine Alkoxy- oder Alkylthiogruppe mit jeweils 2 oder 3 Kohlenstoffatomen im Alkylteil, eine Cyclopropyl- oder Cyclobutylgruppe und

$R_d$ eine in vivo in eine Carboxygruppe überführbare Gruppe, eine Carboxy-, Cyano-, 1H-Tetrazolyl-, 1-Triphenylmethyl-te-trazolyl- oder 2-Triphenylmethyl-tetrazolylgruppe bedeuten,

deren 1-, 3-Isomerengemische und deren Salze.

**2.** Benzimidazole der allgemeinen Formel I gemäß Anspruch 1, in der

$R_a$ bis $R_c$ wie eingangs definiert sind und

$R_d$ eine 1H-Tetrazolylgruppe, eine Carboxygruppe oder eine Gruppe der Formeln

- CO - OR',
- CO - O - (HCR'') - O - CO - R''' und
- CO - O - (HCR'') - O - CO - OR'''

in denen

R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,

R'' ein Wasserstoffatom oder eine Methylgruppe und

R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten,

deren 1-, 3-Isomerengemische und deren Salze.

**3.** Benzimidazole der allgemeinen Formel I gemäß Anspruch 1, in der

$R_a$ eine Methylgruppe, ein Wasserstoff-, Fluor-, Chlor- oder Bromatom,

$R_b$ eine $R_1$NH-Gruppe, in der das Wasserstoffatom durch eine R-CX-Gruppe, durch eine in 2-Stellung durch eine Alkoxygruppe mit 1 bis 3 Kohlenstoffatomen oder durch eine ($R_1NR_3$)-Gruppe substituierte 3,4-Dioxo-1-cyclobuten-1-yl-Gruppe oder durch eine 3-Nitro-pyrrol-2-yl-Gruppe ersetzt ist, wobei die Pyrrolgruppe in 1-Stellung zusätzlich durch eine Methylgruppe substituiert sein kann, in denen

R eine Alkylgruppe mit 1 bis 3 Kohlenstoffatomen, eine Methoxy-, Methylthio-, Phenoxy- oder Phenylthiogruppe oder eine ($R_2NR_3$)-Gruppe und

X eine $NO_2$-CH =, $(NC)_2C$ = oder $R_6$-N = Gruppe, in denen

$R_1$ ein Wasserstoffatom oder eine Alkylgruppe mit 1 bis 3 Kohlenstoffatomen,

$R_2$ eine Alkylgruppe mit 1 bis 3 Kohlenstoffatomen, die in 2- oder 3-Stellung durch eine Hydroxy-, Alkoxy-, Amino-, Alkylamino- oder Dialkylaminogruppe substituiert sein kann, wobei der Alkoxy- und Alkylteil jeweils 1 bis 3 Kohlenstoffatome enthalten kann, oder eine Cycloalkylgruppe mit 5 bis 7 Kohlenstoffatomen,

$R_3$ ein Wasserstoffatom, eine Alkylgruppe mit 1 bis 3 Kohlenstoffatomen, eine 2-Methoxyethyl-, 2-Dimethylaminoethyl- oder 2-Diethylaminoethylgruppe oder

$R_2$ und $R_3$ zusammen mit dem dazwischenliegenden Stickstoffatom eine cyclische Alkyleniminogruppe mit 4 oder 5 Kohlenstoffatomen, die durch ein oder zwei Methylgruppen substituiert sein kann, eine Morpholinogruppe oder eine gegebenenfalls in 4-Stellung durch eine Alkylgruppe mit 1 bis 3 Kohlenstoffatome substituierte Piperazinogruppe und

$R_6$ eine Alkylsulfonylgruppe mit 1 bis 3 Kohlenstoffatomen im Alkylteil, eine Cyano-, Aminosulfonyl- oder Phenylsulfonylgruppe darstellen,

$R_c$ eine Alkylgruppe mit 2 bis 4 Kohlenstoffatomen und

$R_d$ eine Carboxy-, 1H-Tetrazolyl-, 1-Triphenylmethyl-te-trazolyl- oder 2-Triphenylmethyl-tetrazolylgruppe bedeuten,

deren 1-, 3-Isomerengemische und deren Salze.

**4.** Benzimidazole der allgemeinen Formel I gemäß Anspruch 1, in der

$R_a$ in 4-Stellung eine Methylgruppe oder ein Chloratom,

$R_b$ in 6-Stellung eine $R_1NH$-Gruppe, in der das Wasserstoffatom durch eine R-CX-Gruppe, durch eine in 2-Stellung durch eine ($R_1NR_3$)-Gruppe substituierte 3,4-Dioxo-1-cyclobuten-1-yl-Gruppe oder durch eine 3-Nitro-pyrrol-2-yl-Gruppe ersetzt ist, wobei die Pyrrolgruppe in 1-Stellung zusätzlich durch eine Methylgruppe substituiert sein kann, in denen

R eine ($R_2NR_3$)-Gruppe oder eine Alkylgruppe mit 1 bis 3 Kohlenstoffatomen und

X eine $NO_2$-CH=, $(NC)_2C$= oder $R_6$-N= Gruppe, in denen

$R_1$ ein Wasserstoffatom oder eine Methylgruppe,

$R_2$ eine Alkylgruppe mit 1 bis 3 Kohlenstoffatomen, eine 2-Hydroxyethyl-, 2-Methoxyethyl-, 2-Dimethylaminoethyl- oder 2-Diethylaminoethylgruppe oder eine Cycloalkylgruppe mit 5 bis 7 Kohlenstoffatomen,

$R_3$ ein Wasserstoffatom, eine Alkylgruppe mit 1 bis 3 Kohlenstoffatomen, eine 2-Methoxyethyl-, 2-Dimethylaminoethyl- oder 2-Diethylaminoethylgruppe oder

$R_2$ und $R_3$ zusammen mit dem dazwischenliegenden Stickstoffatom eine Pyrrolidino-, Piperidino-, Morpholino- oder 4-Methyl-piperazinogruppe und

$R_6$ eine Cyano-, Aminosulfonyl-, Methylsulfonyl- oder Phenylsulfonylgruppe darstellen,

$R_c$ eine Alkylgruppe mit 2 bis 4 Kohlenstoffatomen und

$R_d$ eine Carboxy- oder 1H-Tetrazolylgruppe bedeuten,

deren 1-, 3-Isomerengemische und deren Salze.

**5.** Folgende Benzimidazole der allgemeinen Formel I gemäß Anspruch 1:

(a) 4'-[[2-n-Propyl-4-methyl-6-(2-cyano-3-methyl-guanidino)-1H-benzimidazol-1-yl)-methyl]-biphenyl-2-carbonsäure,

(b) 4'-[[2-n-Propyl-4-methyl-6-(2-cyano-3,3-dimethyl-guanidino)-1H-benzimidazol-1-yl]-methyl]-2-(1H-tetrazol-5-yl)-biphenyl,

(c) 4'-[[2-n-Propyl-4-methyl-6-(2,2-dicyano-1-dimethylaminoethenylenamino)-1H-benzimidazol-1-yl]-methyl]-biphenyl-2-carbonsäure,

(d) 4'-[[2-n-Propyl-4-methyl-6-(1-dimethylamino-2-nitroethenylenamino)-1H-benzimidazol-1-yl]-methyl]-biphenyl-2-carbonsäure,

(e) 4'-[[2-n-Propyl-4-methyl-6-(3-methyl-2-amidosulfonylguanidino)-1H-benzimidazol-1-yl]-methyl]-biphenyl-2-carbonsäure,

(f) 4'-[[2-n-Propyl-4-methyl-6-(1,1-dimethylamino-2-nitroethylenamino)-1H-benzimidazol-1-yl]-methyl]-2-(1H-tetrazol-5-yl)-biphenyl,

(g) 4'-[[2-Ethyl-4-methyl-6-[(2-dimethylamino-3,4-dioxo-1-cyclobuten-1-yl)-amino]-1H-benzimidazol-1-yl]-methyl]-biphenyl-2-carbonsäure und

(h) 4'-[[2-Ethyl-4-methyl-6-(3,3-dimethyl-2-methylsulfonyl-guanidino)-1H-benzimidazol-1-yl]-methyl]-2-(1H-tetrazol-5-yl)-biphenyl,

deren 1-, 3-Isomerengemische und deren Salze.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels mit Angiotensin-antagonistischer Wirkung.

9. Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

10. Verfahren zur Herstellung der Benzimidazole gemäß den Ansprüchen 1 bis 6, dadurch gekennzeichnet, daß

a) zur Herstellung von Verbindungen der allgemeinen Formel I, in der R eine Alkoxy- oder Alkylthiogruppe mit jeweils 1 bis 3 Kohlenstoffatomen oder eine Phenyloxy- oder Phenylthiogruppe darstellt, eine Verbindung der allgemeinen Formel

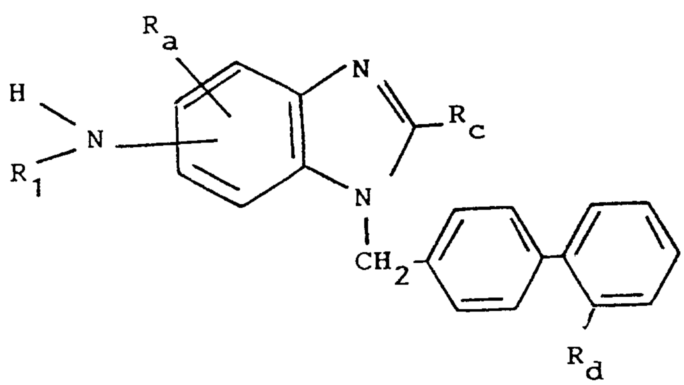

,(II)

in der

$R_a$, $R_c$, $R_d$ und $R_1$ wie in den Ansprüchen 1 bis 5 definiert sind, mit einer Verbindung der allgemeinen Formel

$(Y')_2C=X$ ,(III)

in der

X wie in den Ansprüchen 1 bis 5 definiert ist,

die Reste Y', die gleich oder verschieden sein können, jeweils eine Alkoxy- oder Alkylthiogruppe mit jeweils 1 bis 3 Kohlenstoffatomen, eine Phenoxy- oder Phenylthiogruppe darstellen, umgesetzt wird oder

b) zur Herstellung von Verbindungen der allgemeinen Formel I, in der R eine $(R_2NR_3)$-Gruppe darstellt, eine Verbindung der allgemeinen Formel

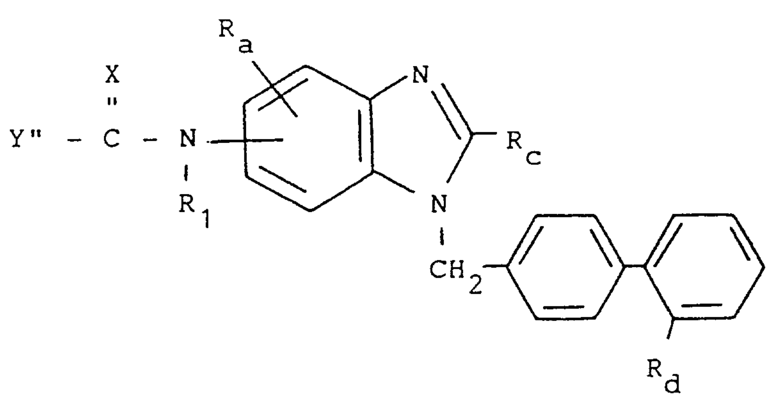

,(IV)

in der

$R_a$, $R_c$, $R_d$, $R_1$ und X wie in den Ansprüchen 1 bis 5 definiert sind und

Y'' eine Alkoxy-, Alkylthio-, Alkylsulfinyl- oder Alkylsulfonylgruppe mit jeweils 1 bis 3 Kohlenstoffatomen, eine Phenyloxy-, Phenylthio-, Phenylsulfinyl- oder Phenylsulfonylgruppe darstellt, mit einem Amin der allgemeinen Formel

$R_2R_3NH$ ,(V)

in der

$R_2$ und $R_3$ wie in den Ansprüchen 1 bis 5 definiert sind, umgesetzt wird oder

c) zur Herstellung von Verbindungen der allgemeinen Formel I, in der R eine Alkylgruppe mit 1 bis 5 Kohlenstoffatomen darstellt, eine Verbindung der allgemeinen Formel

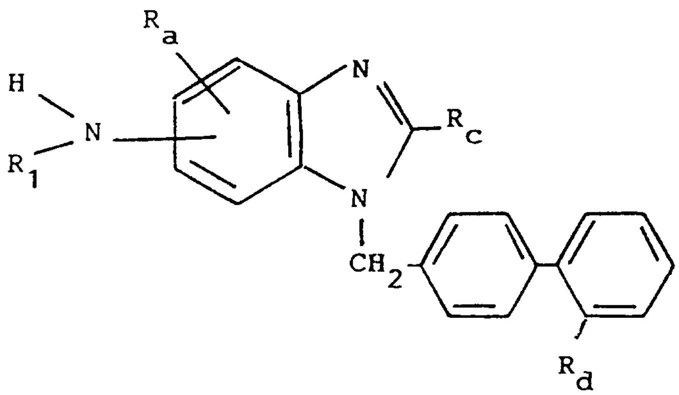

,(II)

in der

$R_a$, $R_c$, $R_d$ und $R_1$ wie in den Ansprüchen 1 bis 5 definiert sind, mit einer Verbindung der allgemeinen Formel

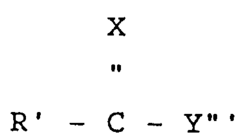

,(VI)

in der

X wie in den Ansprüchen 1 bis 5 definiert ist,

R' eine Alkylgruppe mit 1 bis 5 Kohlenstoffatomen und

Y''' eine Alkylthiogruppe mit 1 bis 3 Kohlenstoffatomen darstellt, umgesetzt wird oder

d) zur Herstellung einer Verbindung der allgemeinen Formel I, in der $R_d$ eine Carboxygruppe darstellt, eine Verbindung der allgemeinen Formel

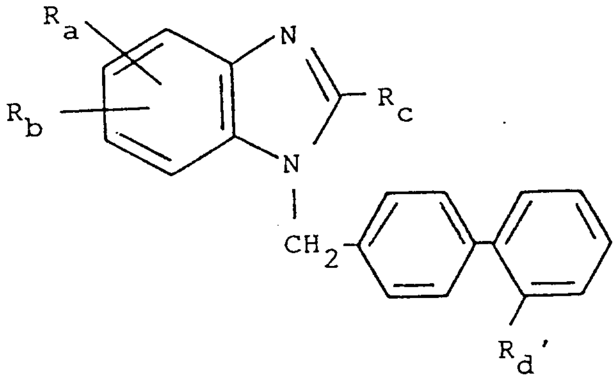

,(VII)

in der
$R_a$ bis $R_c$ wie in den Ansprüchen 1 bis 5 definiert sind und
$R_d$' eine mittels Hydrolyse, Thermolyse oder Hydrogenolyse in eine Carboxygruppe überführbare Gruppe darstellt, in eine entsprechende Carboxyverbindung übergeführt wird oder
e) zur Herstellung einer Verbindung der allgemeinen Formel I, in der $R_d$ eine 1H-Tetrazolylgruppe darstellt, ein Schutzrest von einer Verbindung der allgemeinen Formel

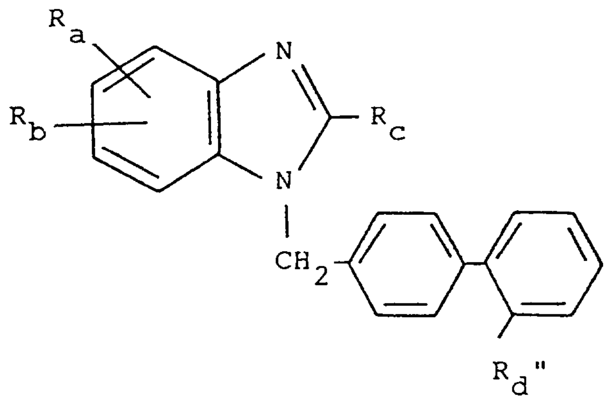

,(VIII)

in der
$R_a$ bis $R_c$ wie in den Ansprüchen 1 bis 5 definiert sind und
$R_d$'' eine in 1- oder 2-Stellung durch einen Schutzrest geschützte 1H-Tetrazolyl- oder 2H-Tetrazolylgruppe darstellt, abgespalten wird oder
f) zur Herstellung von Verbindungen der allgemeinen Formel I, in der $R_b$ eine $R_1$NH-Gruppe darstellt, in der das Wasserstoffatom durch eine der eingangs erwähnten 3-Nitro-pyrrol-2-yl-Reste ersetzt ist, eine gegebenenfalls im Reaktionsgemisch gebildete Verbindung der allgemeinen Formel

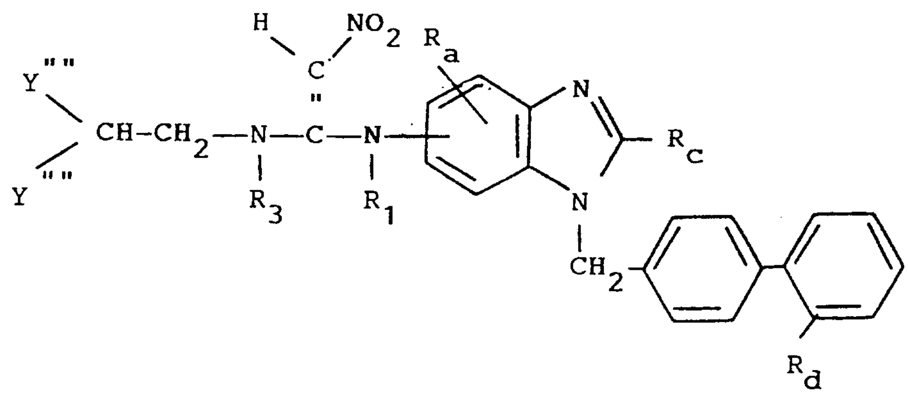

,(IX)

in der
$R_1$, $R_3$, $R_a$, $R_c$ und $R_d$ wie in den Ansprüchen 1 bis 5 definiert sind und
die Reste Y'''', die gleich oder verschieden sein können, jeweils eine Alkoxygruppe mit 1 bis 3 Kohlenstoffatomen darstellen, cyclisiert wird oder
g) zur Herstellung von Verbindungen der allgemeinen Formel I, in der $R_b$ eine $R_1$NH-Gruppe darstellt, in der das Wasserstoffatom durch eine der eingangs erwähnten 3,4-Dioxo-1-cyclobuten-1-yl-Reste ersetzt ist, eine Verbindung der allgemeinen Formel

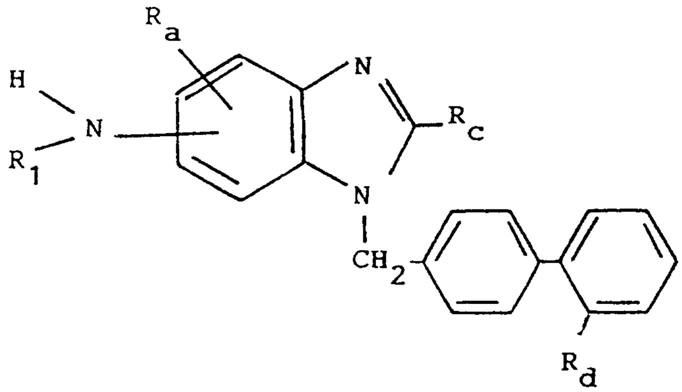

,(II)

in der $R_a$, $R_c$, $R_d$ und $R_1$ wie in den Ansprüchen 1 bis 5 definiert sind, mit einem 3,4-Dioxo-1,3-dialkoxy-1-cyclobuten mit jeweils 1 bis 3 Kohlenstoffatomen im Alkoxyteil umgesetzt und
zur Herstellung einer entsprechenden 2-Aminoverbindung anschließend eine der so erhaltene 2-Alkoxyverbindung mit einem Amin der allgemeinen Formel

$R_2R_3NH$ ,(V)

in der
$R_2$ und $R_3$ wie in den Ansprüchen 1 bis 5 definiert sind, umgesetzt wird oder
h) zur Herstellung einer Verbindung der allgemeinen Formel I, in der $R_d$ eine 1H-Tetrazolylgruppe darstellt, eine Verbindung der allgemeinen Formel

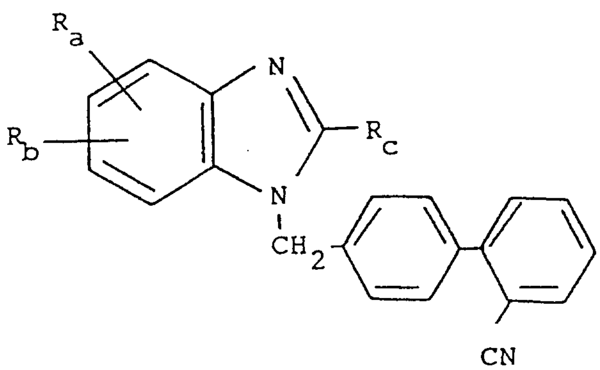

, (X)

in der
$R_a$ bis $R_c$ wie in den Ansprüchen 1 bis 5 definiert sind, mit Stickstoffwasserstoffsäure oder deren Salzen umgesetzt wird und
erforderlichenfalls ein während der Umsetzungen a) bis e) zum Schutze von reaktiven Gruppen verwendeter Schutzrest abgespalten wird und/oder
gewünschtenfalls anschließend ein so erhaltenes 1-, 3-Isomerengemisch einer Verbindung der allgemeinen Formel I mittels Isomerentrennung in ihr 1- und 3-Isomer aufgetrennt wird oder
eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.